# EUROPEAN PATENT SPECIFICATION

(11) **EP 1 026 238 B1**
(45) Date of publication and mention of the grant of the patent: **28.02.2007**
(21) Application number: 00102229.2
(22) Date of filing: 17.06.1992
(51) Int. Cl.: C12N 9/88, C07K 16/40, C12N 15/60, A61K 38/51, G01N 33/573

(54) **Cloned glutamic acid decarboxylase**
Klonierte Glutaminsäure-Decarboxylase
Décarboxylase de l'acide glutamique cloné

(30) Priority: 18.06.1991 US 716909
(43) Date of publication of application: 09.08.2000
(62) Divisional of application: 92110308.1
(73) Proprietor: The Regents of the University of California, Oakland, California 94612-3550 (US)
(72) Inventor: Tobin, Allan J., Los Angeles, California 90036 (US); Erlander, Mark G., R.W. Johnson Pharma., San Diego, CA 92121 (US); Kaufman, Daniel L., Santa Monica, California 90404 (US); Clare-Salzler, Michael J., Los Angeles, California 90068 (US)
(74) Representative: Vossius & Partner

(56) References cited:
- BAEKKESKOV E.A.: "Identification of the 64K autoantigen in insulin-dependent diabetes as the GABA-synthesizing enzyme glutamic acid decarboxylase" NATURE, vol. 347, 1990, pages 151-156, XP002141580 LONDON GB

### SEQUENCE LISTING

<110> The Regents of the University of California
<120> Cloned Glutamic Acid Decarboxylase
<130> C 773 EP/I
<140>
   <141>
<160> 9
<170> PatentIn Ver. 2.1
<210> 1
   <211> 24
   <212> PRT
   <213> Homo sapiens
<400> 1
   Ser Ile Met Ala Ala Arg Tyr Lys Tyr Phe Pro Glu Val Lys Thr Lys
     1               5                  10                  15
   Gly Met Ala Ala Val Pro Lys Leu
                20
<210> 2
   <211> 24
   <212> PRT
   <213> Homo sapiens
<400> 2
   Ala Met Met Ile Ala Arg Phe Lys Met Phe Pro Glu Val Lys Glu Lys
    1               5                  10                  15
   Gly Met Ala Ala Leu Pro Arg Leu
               20
<210> 3
   <211> 23
   <212> PRT
   <213> Coxsackievirus
<400> 3
   Phe Ile Glu Trp Leu Lys Val Lys Ile Leu Pro Glu Val Lys Glu Lys
    1               5                  10                  15
   His Glu Phe Leu Ser Arg Leu
               20
<210> 4
   <211> 15
   <212> PRT
   <213> Poliovirus
<400> 4
   Met Lys Ser Met Cys Pro Gln Ala Gln Leu Lys Val Lys Tyr Leu
    1               5                  10                  15
<210> 5
   <211> 60
   <212> PRT
   <213> Felis catus
<400> 5
   Tyr Glu Ile Ala Pro Val Phe Val Leu Met Glu Gln Ile Thr Leu Lys
    1               5                  10                  15
   Lys Met Arg Glu Ile Val Gly Trp Ser Ser Lys Asp Gly Asp Gly Ile
               20                  25                  30
   Phe Ser Pro Gly Gly Ala Ile Ser Asn Met Tyr Ser Ile Met Ala Ala
           35                  40                  45
   Arg Tyr Lys Phe Phe Pro Glu Val Lys Thr Lys Gly
       50                  55                  60
<210> 6
   <211> 1966
   <212> DNA
   <213> Rattus rattus
<220>
   <221> CDS
   <222> (75)..(1829)
<400> 6
   gggcgtgcgg ggtcgagccg aagcagcttg cccgcagcca ctcggaggcg accagcgcca 60
   gactagcaga accc atg gca tct ccg ggc tct ggc ttt tgg tcc ttc gga   110
                   Met Ala Ser Pro Gly Ser Gly Phe Trp Ser Phe Gly
                   1               5                   10
   tct gaa gat ggc tct ggg gat cct gag aac ccg gga aca gcg aga gcc   158
   Ser Glu Asp Gly Ser Gly Asp Pro Glu Asn Pro Gly Thr Ala Arg Ala
           15                  20                  25
   tgg tgc cag gtg gcc caa aag ttc acg ggc ggc atc gga aac aag cta   206
   Trp Cys Gln Val Ala Gln Lys Phe Thr Gly Gly Ile Gly Asn Lys Leu
       30                  35                  40
   tgc gct ctg ctc tac gga gac tct gag aag cca gca gag agc ggc ggg   254
   Cys Ala Leu Leu Tyr Gly Asp Ser Glu Lys Pro Ala Glu Ser Gly Gly
   45                  50                  55                  60
   agc gtg acc tcg cgg gcc gcc act cgg aag gtc gcc tgc acc tgt gac   302
   Ser Val Thr Ser Arg Ala Ala Thr Arg Lys Val Ala Cys Thr Cys Asp
                   65                  70                  75
   caa aaa ccc tgc agc tgc ccc aaa gga gat gtc aat tat gca ctt ctc   350
   Gln Lys Pro Cys Ser Cys Pro Lys Gly Asp Val Asn Tyr Ala Leu Leu
               80                  85                  90
   cac gca aca gac ctg ctg cca gcc tgt gaa gga gaa agg ccc act ctc   398
   His Ala Thr Asp Leu Leu Pro Ala Cys Glu Gly Glu Arg Pro Thr Leu
           95                  100                 105
   gca ttt ctg caa gat gta atg aac att ttg ctt cag tac gtg gtg aaa   446
   Ala Phe Leu Gln Asp Val Met Asn Ile Leu Leu Gln Tyr Val Val Lys
       110                 115                 120
   agt ttt gat aga tca act aaa gtg att gat ttc cat tac ccc aat gag    494
   Ser Phe Asp Arg Ser Thr Lys Val Ile Asp Phe His Tyr Pro Asn Glu
   125                 130                 135                 140
   ctt ctt caa gag tat aat tgg gaa ttg gca gac caa ccg caa aat ctg    542
   Leu Leu Gln Glu Tyr Asn Trp Glu Leu Ala Asp Gln Pro Gln Asn Leu
                   145                 150                 155
   gag gaa att ttg acg cac tgc caa aca act cta aaa tat gcg att aaa    590
   Glu Glu Ile Leu Thr His Cys Gln Thr Thr Leu Lys Tyr Ala Ile Lys
               160                 165                 170
   aca ggg cat ccc cga tat ttt aat cag ctg tct acc gga ttg gat atg    638
   Thr Gly His Pro Arg Tyr Phe Asn Gln Leu Ser Thr Gly Leu Asp Met
           175                 180                 185
   gtt gga tta gca gca gat tgg ttg aca tca aca gca aac acg aac atg    686
   Val Gly Leu Ala Ala Asp Trp Leu Thr Ser Thr Ala Asn Thr Asn Met
       190                 195                 200
   ttt acc tat gag atc gcc cct gta ttt gta cta ctg gaa tat gtg aca    734
   Phe Thr Tyr Glu Ile Ala Pro Val Phe Val Leu Leu Glu Tyr Val Thr
   205                 210                 215                 220
   cta aag aaa atg agg gaa atc att ggc tgg cca gga ggc tct ggc gat    782
   Leu Lys Lys Met Arg Glu Ile Ile Gly Trp Pro Gly Gly Ser Gly Asp
                   225                 230                 235
   gga atc ttt tct cct ggt ggt gcc atc tcc aac atg tac gcc atg ctc    830
   Gly Ile Phe Ser Pro Gly Gly Ala Ile Ser Asn Met Tyr Ala Met Leu
               240                 245                 250
   att gcc cgc tat aag atg ttt cca gaa gtc aag gaa aag ggg atg gcg    878
   Ile Ala Arg Tyr Lys Met Phe Pro Glu Val Lys Glu Lys Gly Met Ala
           255                 260                 265
   gcg gtg ccc agg ctc atc gca ttc acg tca gag cat agt cac ttt tct    926
   Ala Val Pro Arg Leu Ile Ala Phe Thr Ser Glu His Ser His Phe Ser
       270                 275                 280
   ctc aag aag gga gct gca gcc ttg ggg atc gga aca gac agc gtg att    974
   Leu Lys Lys Gly Ala Ala Ala Leu Gly Ile Gly Thr Asp Ser Val Ile
   285                 290                 295                 300
   ctg att aaa tgt gat gag aga ggg aaa atg atc cca tct gac ctt gaa    1022
   Leu Ile Lys Cys Asp Glu Arg Gly Lys Met Ile Pro Ser Asp Leu Glu
                   305                 310                 315
   aga aga atc ctt gaa gtc aaa cag aaa gga ttt gtt cct ttc ctg gtg    1070
   Arg Arg Ile Leu Glu Val Lys Gln Lys Gly Phe Val Pro Phe Leu Val
               320                 325                 330
   agt gcc aca gct gga acc act gtg tac ggg gct ttt gat cct ctc ttg    1118
   Ser Ala Thr Ala Gly Thr Thr Val Tyr Gly Ala Phe Asp Pro Leu Leu
           335                 340                 345
   gct gta gct gac atc tgc aaa aaa tat aag atc tgg atg cat gtg gat    1166
   Ala Val Ala Asp Ile Cys Lys Lys Tyr Lys Ile Trp Met His Val Asp
       350                 355                 360
   gct gct tgg ggt gga ggg tta ctg atg tct cgg aaa cac aag tgg aag    1214
   Ala Ala Trp Gly Gly Gly Leu Leu Met Ser Arg Lys His Lys Trp Lys
   365                 370                 375                 380
   ctg aac ggt gtg gag agg gcc aac tct gtg aca tgg aat ccc cac aag   1262
   Leu Asn Gly Val Glu Arg Ala Asn Ser Val Thr Trp Asn Pro His Lys
                   385                 390                 395
   atg atg ggt gtc ccc ttg caa tgt tcg gct ctc ctg gtc aga gag gag   1310
   Met Met Gly Val Pro Leu Gln Cys Ser Ala Leu Leu Val Arg Glu Glu
               400                 405                 410
   gga ctg atg cag agc tgc aac cag atg cat gct tcc tac ctc ttt cag   1358
   Gly Leu Met Gln Ser Cys Asn Gln Met His Ala Ser Tyr Leu Phe Gln
           415                 420                 425
   caa gat aag cac tat gac ctg tcc tat gac acg gga gac aag gcc ttg   1406
   Gln Asp Lys His Tyr Asp Leu Ser Tyr Asp Thr Gly Asp Lys Ala Leu
       430                 435                 440
   cag tgt gga cgc cac gtc gat gtc ttt aaa tta tgg ctc atg tgg aga   1454
   Gln Cys Gly Arg His Val Asp Val Phe Lys Leu Trp Leu Met Trp Arg
   445                 450                 455                 460
   gca aag ggg act act gga ttt gaa gct cac att gat aag tgt ttg gag   1502
   Ala Lys Gly Thr Thr Gly Phe Glu Ala His Ile Asp Lys Cys Leu Glu
                   465                 470                 475
   ctg gca gag tat tta tac aat atc att aaa aac cga gaa gga tat gaa   1550
   Leu Ala Glu Tyr Leu Tyr Asn Ile Ile Lys Asn Arg Glu Gly Tyr Glu
               480                 485                 490
   atg gtg ttc gat ggg aag cct cag cac aca aat gtc tgc ttc tgg ttt   1598
   Met Val Phe Asp Gly Lys Pro Gln His Thr Asn Val Cys Phe Trp Phe
           495                 500                 505
   gta cct cct agt ttg cga gtt ctg gaa gac aat gaa gag aga atg agc   1646
   Val Pro Pro Ser Leu Arg Val Leu Glu Asp Asn Glu Glu Arg Met Ser
       510                 515                 520
   cgc ctc tca aag gtg gcg cca gtg att aaa gcc aga atg atg gag tat   1694
   Arg Leu Ser Lys Val Ala Pro Val Ile Lys Ala Arg Met Met Glu Tyr
   525                 530                 535                 540
   ggg acc aca atg gtc agc tac caa ccc tta gga gat aag gtc aac ttc   1742
   Gly Thr Thr Met Val Ser Tyr Gln Pro Leu Gly Asp Lys Val Asn Phe
                   545                 550                 555
   ttc cgc atg gtc atc tca aac cct gca gca act cac caa gac att gac   1790
   Phe Arg Met Val Ile Ser Asn Pro Ala Ala Thr His Gln Asp Ile Asp
               560                 565                 570
   ttc ctc att gaa gaa atc gaa cgc ctg gga caa gat ttg taatcacttt    1839
   Phe Leu Ile Glu Glu Ile Glu Arg Leu Gly Gln Asp Leu
           575                 580                 585
   gctcaccaaa ctttcagttc tctaggtaga cagctaagtt gtcacaaact gtgtaaatgt 1899
   atttgtagtt tgttccagag taattctatt tctatatcgt ggtgtcacag tagagtccag 1959
   tttaaaa                                                           1966
<210> 7
   <211> 585
   <212> PRT
   <213> Rattus rattus
<400> 7
   Met Ala Ser Pro Gly Ser Gly Phe Trp Ser Phe Gly Ser Glu Asp Gly
   1 5 10 15
   Ser Gly Asp Pro Glu Asn Pro Gly Thr Ala Arg Ala Trp Cys Gln Val
   20 25 30
   Ala Gln Lys Phe Thr Gly Gly Ile Gly Asn Lys Leu Cys Ala Leu Leu
   35 40 45
   Tyr Gly Asp Ser Glu Lys Pro Ala Glu Ser Gly Gly Ser Val Thr Ser
   50 55 60
   Arg Ala Ala Thr Arg Lys Val Ala Cys Thr Cys Asp Gln Lys Pro Cys
   65 70 75 80
   Ser Cys Pro Lys Gly Asp Val Asn Tyr Ala Leu Leu His Ala Thr Asp
   85 90 95
   Leu Leu Pro Ala Cys Glu Gly Glu Arg Pro Thr Leu Ala Phe Leu Gln
   100 105 110
   Asp Val Met Asn Ile Leu Leu Gln Tyr Val Val Lys Ser Phe Asp Arg
   115 120 125
   Ser Thr Lys Val Ile Asp Phe His Tyr Pro Asn Glu Leu Leu Gln Glu
   130 135 140
   Tyr Asn Trp Glu Leu Ala Asp Gln Pro Gln Asn Leu Glu Glu Ile Leu
   145 150 155 160
   Thr His Cys Gln Thr Thr Leu Lys Tyr Ala Ile Lys Thr Gly His Pro
   165 170 175
   Arg Tyr Phe Asn Gln Leu Ser Thr Gly Leu Asp Met Val Gly Leu Ala
   180 185 190
   Ala Asp Trp Leu Thr Ser Thr Ala Asn Thr Asn Met Phe Thr Tyr Glu
   195 200 205
   Ile Ala Pro Val Phe Val Leu Leu Glu Tyr Val Thr Leu Lys Lys Met
   210 215 220
   Arg Glu Ile Ile Gly Trp Pro Gly Gly Ser Gly Asp Gly Ile Phe Ser
   225 230 235 240
   Pro Gly Gly Ala Ile Ser Asn Met Tyr Ala Met Leu Ile Ala Arg Tyr
   245 250 255
   Lys Met Phe Pro Glu Val Lys Glu Lys Gly Met Ala Ala Val Pro Arg
   260 265 270
   Leu Ile Ala Phe Thr Ser Glu His Ser His Phe Ser Leu Lys Lys Gly
   275 280 285
   Ala Ala Ala Leu Gly Ile Gly Thr Asp Ser Val Ile Leu Ile Lys Cys
   290 295 300
   Asp Glu Arg Gly Lys Met Ile Pro Ser Asp Leu Glu Arg Arg Ile Leu
   305 310 315 320
   Glu Val Lys Gln Lys Gly Phe Val Pro Phe Leu Val Ser Ala Thr Ala
                   325                 330                 335
   Gly Thr Thr Val Tyr Gly Ala Phe Asp Pro Leu Leu Ala Val Ala Asp
               340                 345                 350
   Ile Cys Lys Lys Tyr Lys Ile Trp Met His Val Asp Ala Ala Trp Gly
           355                 360                 365
   Gly Gly Leu Leu Met Ser Arg Lys His Lys Trp Lys Leu Asn Gly Val
       370                 375                 380
   Glu Arg Ala Asn Ser Val Thr Trp Asn Pro His Lys Met Met Gly Val
   385                 390                 395                 400
   Pro Leu Gln Cys Ser Ala Leu Leu Val Arg Glu Glu Gly Leu Met Gln
                   405                 410                 415
   Ser Cys Asn Gln Met His Ala Ser Tyr Leu Phe Gln Gln Asp Lys His
               420                 425                 430
   Tyr Asp Leu Ser Tyr Asp Thr Gly Asp Lys Ala Leu Gln Cys Gly Arg
           435                 440                 445
   His Val Asp Val Phe Lys Leu Trp Leu Met Trp Arg Ala Lys Gly Thr
       450                 455                 460
   Thr Gly Phe Glu Ala His Ile Asp Lys Cys Leu Glu Leu Ala Glu Tyr
   465                 470                 475                 480
   Leu Tyr Asn Ile Ile Lys Asn Arg Glu Gly Tyr Glu Met Val Phe Asp
                   485                 490                 495
   Gly Lys Pro Gln His Thr Asn Val Cys Phe Trp Phe Val Pro Pro Ser
               500                 505                 510
   Leu Arg Val Leu Glu Asp Asn Glu Glu Arg Met Ser Arg Leu Ser Lys
           515                 520                 525
   Val Ala Pro Val Ile Lys Ala Arg Met Met Glu Tyr Gly Thr Thr Met
       530                 535                 540
   Val Ser Tyr Gln Pro Leu Gly Asp Lys Val Asn Phe Phe Arg Met Val
   545                 550                 555                 560
   Ile Ser Asn Pro Ala Ala Thr His Gln Asp Ile Asp Phe Leu Ile Glu
                   565                 570                 575
   Glu Ile Glu Arg Leu Gly Gln Asp Leu
               580                 585
<210> 8
   <211> 2400
   <212> DNA
   <213> Homo sapiens
<220>
   <221> CDS
   <222> (53)..(1810)
<400> 8
   agctcgcccg cagctcgcac tcgcaggcga cctgctccag tctccaaagc cg atg gca 58
                                                             Met Ala
                                                               1
   tct ccg ggc tct ggc ttt tgg tct ttc ggg tcg gaa gat ggc tct ggg   106
   Ser Pro Gly Ser Gly Phe Trp Ser Phe Gly Ser Glu Asp Gly Ser Gly
           5                  10                  15
   gat tcc gag aat ccc ggc aca gcg cga gcc tgg tgc caa gtg gct cag   154
   Asp Ser Glu Asn Pro Gly Thr Ala Arg Ala Trp Cys Gln Val Ala Gln
       20                  25                  30
   aag ttc acg ggc ggc atc gga aac aaa ctg tgc gcc ctg ctc tac gga   202
   Lys Phe Thr Gly Gly Ile Gly Asn Lys Leu Cys Ala Leu Leu Tyr Gly
    35                  40                  45                  50
   gac gcc gag aag ccg gcg gag agc ggc ggg agc caa ccc ccg cgg gcc   250
   Asp Ala Glu Lys Pro Ala Glu Ser Gly Gly Ser Gln Pro Pro Arg Ala
                   55                  60                  65
   gcc gcc cgg aag gcc gcc tgc gcc tgc gac cag aag ccc tgc agc tgc   298
   Ala Ala Arg Lys Ala Ala Cys Ala Cys Asp Gln Lys Pro Cys Ser Cys
               70                  75                  80
   tcc aaa gtg gat gtc aac tac gcg ttt ctc cat gca aca gac ctg ctg   346
   Ser Lys Val Asp Val Asn Tyr Ala Phe Leu His Ala Thr Asp Leu Leu
           85                  90                  95
   ccg gcg tgt gat gga gaa agg ccc act ttg gcg ttt ctg caa gat gtt   394
   Pro Ala Cys Asp Gly Glu Arg Pro Thr Leu Ala Phe Leu Gln Asp Val
       100                 105                 110
   atg aac att tta ctt cag tat gtg gtg aaa agt ttc gat aga tca acc   442
   Met Asn Ile Leu Leu Gln Tyr Val Val Lys Ser Phe Asp Arg Ser Thr
   115                 120                 125                 130
   aaa gtg att gat ttc cat tat cct aat gag ctt ctc caa gaa tat aat   490
   Lys Val Ile Asp Phe His Tyr Pro Asn Glu Leu Leu Gln Glu Tyr Asn
                   135                 140                 145
   tgg gaa ttg gca gac caa cca caa aat ttg gag gaa att ttg atg cat   538
   Trp Glu Leu Ala Asp Gln Pro Gln Asn Leu Glu Glu Ile Leu Met His
               150                 155                 160
   tgc caa aca act cta aaa tat gca att aaa aca ggg cat cct aga tac   586
   Cys Gln Thr Thr Leu Lys Tyr Ala Ile Lys Thr Gly His Pro Arg Tyr
           165                 170                 175
   ttc aat caa ctt tct act ggt ttg gat atg gtt gga tta gca gca gac   634
   Phe Asn Gln Leu Ser Thr Gly Leu Asp Met Val Gly Leu Ala Ala Asp
       180                 185                 190
   tgg ctg aca tca aca gca aat act aac atg ttc acc tat gaa att gct   682
   Trp Leu Thr Ser Thr Ala Asn Thr Asn Met Phe Thr Tyr Glu Ile Ala
   195                 200                 205                 210
   cca gta ttt gtg ctt ttg gaa tat gtc aca cta aag aaa atg aga gaa   730
   Pro Val Phe Val Leu Leu Glu Tyr Val Thr Leu Lys Lys Met Arg Glu
                   215                 220                 225
   atc att ggc tgg cca ggg ggc tct ggc gat ggg ata ttt tct ccc ggt   778
   Ile Ile Gly Trp Pro Gly Gly Ser Gly Asp Gly Ile Phe Ser Pro Gly
                 230                 235                 240
   ggc gcc ata tct aac atg tat gcc atg atg atc gca cgc ttt aag atg    826
   Gly Ala Ile Ser Asn Met Tyr Ala Met Met Ile Ala Arg Phe Lys Met
           245                 250                 255
   ttc cca gaa gtc aag gag aaa gga atg gct gct ctt ccc agg ctc att    874
   Phe Pro Glu Val Lys Glu Lys Gly Met Ala Ala Leu Pro Arg Leu Ile
       260                 265                 270
   gcc ttc acg tct gaa cat agt cat ttt tct ctc aag aag gga gct gca    922
   Ala Phe Thr Ser Glu His Ser His Phe Ser Leu Lys Lys Gly Ala Ala
   275                 280                 285                 290
   gcc tta ggg att gga aca gac agc gtg att ctg att aaa tgt gat gag    970
   Ala Leu Gly Ile Gly Thr Asp Ser Val Ile Leu Ile Lys Cys Asp Glu
                   295                 300                 305
   aga ggg aaa atg att cca tct gat ctt gaa aga agg att ctt gaa gcc   1018
   Arg Gly Lys Met Ile Pro Ser Asp Leu Glu Arg Arg Ile Leu Glu Ala
               310                 315                 320
   aaa cag aaa ggg ttt gtt cct ttc ctc gtg agt gcc aca gct gga acc   1066
   Lys Gln Lys Gly Phe Val Pro Phe Leu Val Ser Ala Thr Ala Gly Thr
           325                 330                 335
   acc gtg tac gga gca ttt gac ccc ctc tta gct gtc gct gac att tgc   1114
   Thr Val Tyr Gly Ala Phe Asp Pro Leu Leu Ala Val Ala Asp Ile Cys
       340                 345                 350
   aaa aag tat aag atc tgg atg cat gtg gat gca gct tgg ggt ggg gga   1162
   Lys Lys Tyr Lys Ile Trp Met His Val Asp Ala Ala Trp Gly Gly Gly
   355                 360                 365                 370
   tta ctg atg tcc cga aaa cac aag tgg aaa ctg agt ggc gtg gag agg   1210
   Leu Leu Met Ser Arg Lys His Lys Trp Lys Leu Ser Gly Val Glu Arg
                   375                 380                 385
   gcc aac tct gtg acg tgg aat cca cac aag atg atg gga gtc cct ttg   1258
   Ala Asn Ser Val Thr Trp Asn Pro His Lys Met Met Gly Val Pro Leu
               390                 395                 400
   cag tgc tct gct ctc ctg gtt aga gaa gag gga ttg atg cag aat tgc   1306
   Gln Cys Ser Ala Leu Leu Val Arg Glu Glu Gly Leu Met Gln Asn Cys
           405                 410                 415
   aac caa atg cat gcc tcc tac ctc ttt cag caa gat aaa cat tat gac   1354
   Asn Gln Met His Ala Ser Tyr Leu Phe Gln Gln Asp Lys His Tyr Asp
       420                 425                 430
   ctg tcc tat gac act gga gac aag gcc tta cag tgc gga cgc cac gtt   1402
   Leu Ser Tyr Asp Thr Gly Asp Lys Ala Leu Gln Cys Gly Arg His Val
   435                 440                 445                 450
   gat gtt ttt aaa cta tgg ctg atg tgg agg gca aag ggg act acc ggg   1450
   Asp Val Phe Lys Leu Trp Leu Met Trp Arg Ala Lys Gly Thr Thr Gly
                   455                 460                 465
   ttt gaa gcg cat gtt gat aaa tgt ttg gag ttg gca gag tat tta tac   1498
   Phe Glu Ala His Val Asp Lys Cys Leu Glu Leu Ala Glu Tyr Leu Tyr
               470                 475                 480
   aac atc ata aaa aac cga gaa gga tat gag atg gtg ttt gat ggg aag   1546
   Asn Ile Ile Lys Asn Arg Glu Gly Tyr Glu Met Val Phe Asp Gly Lys
           485                 490                 495
   cct cag cac aca aat gtc tgc ttc tgg tac att cct cca agc ttg cgt   1594
   Pro Gln His Thr Asn Val Cys Phe Trp Tyr Ile Pro Pro Ser Leu Arg
       500                 505                 510
   act ctg gaa gac aat gaa gag aga atg agt cgc ctc tcg aag gtg gct   1642
   Thr Leu Glu Asp Asn Glu Glu Arg Met Ser Arg Leu Ser Lys Val Ala
   515                 520                 525                 530
   cca gtg att aaa gcc aga atg atg gag tat gga acc aca atg gtc agc   1690
   Pro Val Ile Lys Ala Arg Met Met Glu Tyr Gly Thr Thr Met Val Ser
                   535                 540                 545
   tac caa ccc ttg gga gac aag gtc aat ttc ttc cgc atg gtc atc tca   1738
   Tyr Gln Pro Leu Gly Asp Lys Val Asn Phe Phe Arg Met Val Ile Ser
               550                 555                 560
   aac cca gcg gca act cac caa gac att gac ttc ctg att gaa gaa ata   1786
   Asn Pro Ala Ala Thr His Gln Asp Ile Asp Phe Leu Ile Glu Glu Ile
           565                 570                 575
   gaa cgc ctt gga caa gat tta taa taaccttgct caccaagctg ttccacttct  1840
   Glu Arg Leu Gly Gln Asp Leu
       580                 585
   ctaggtagac aattaagttg tcacaaactg tgtgaatgta tttgtagttt gttccaaagt 1900
   aaatctattt ctatattgtg gtgtcaaagt agagtttaaa aattaaacaa aaaagacatt 1960
   gctcctttta aaagtccttt cttaagttta gaatacctct ctaagaattc gtgacaaaag 2020
   gctatgttct aatcaataag gaaaagctta aaattgttat aaatacttcc cttactttta 2080
   atatagtgtg caaagcaaac tttattttca cttcagacta gtaggactga atagtgccaa 2140
   attgcccctg aatcataaaa ggttctttgg ggtgcagtaa aaaggacaaa gtaaatataa 2200
   aatatatgtt gacaataaaa actcttgcct ttttcatagt attagaaaaa aatttctaat 2260
   ttacctatag caacatttca aatgtattta aatacatata attttacaaa aggaaaatat 2320
   atatattaaa aaagatatcc tattttgtaa catatagatt tttattttat ataggttata 2380
   caaactgcgg gggcggaatt                                             2400
<210> 9
   <211> 585
   <212> PRT
   <213> Homo sapiens
<400> 9
   Met Ala Ser Pro Gly Ser Gly Phe Trp Ser Phe Gly Ser Glu Asp Gly
    1               5                  10                  15
   Ser Gly Asp Ser Glu Asn Pro Gly Thr Ala Arg Ala Trp Cys Gln Val
               20                  25                  30
   Ala Gln Lys Phe Thr Gly Gly Ile Gly Asn Lys Leu Cys Ala Leu Leu
           35                  40                  45
   Tyr Gly Asp Ala Glu Lys Pro Ala Glu Ser Gly Gly Ser Gln Pro Pro
       50                  55                  60
   Arg Ala Ala Ala Arg Lys Ala Ala Cys Ala Cys Asp Gln Lys Pro Cys
   65                  70                  75                  80
   Ser Cys Ser Lys Val Asp Val Asn Tyr Ala Phe Leu His Ala Thr Asp
                   85                  90                  95
   Leu Leu Pro Ala Cys Asp Gly Glu Arg Pro Thr Leu Ala Phe Leu Gln
               100                 105                 110
   Asp Val Met Asn Ile Leu Leu Gln Tyr Val Val Lys Ser Phe Asp Arg
           115                 120                 125
   Ser Thr Lys Val Ile Asp Phe His Tyr Pro Asn Glu Leu Leu Gln Glu
       130                 135                 140
   Tyr Asn Trp Glu Leu Ala Asp Gln Pro Gln Asn Leu Glu Glu Ile Leu
   145                 150                 155                 160
   Met His Cys Gln Thr Thr Leu Lys Tyr Ala Ile Lys Thr Gly His Pro
                   165                 170                 175
   Arg Tyr Phe Asn Gln Leu Ser Thr Gly Leu Asp Met Val Gly Leu Ala
               180                 185                 190
   Ala Asp Trp Leu Thr Ser Thr Ala Asn Thr Asn Met Phe Thr Tyr Glu
           195                 200                 205
   Ile Ala Pro Val Phe Val Leu Leu Glu Tyr Val Thr Leu Lys Lys Met
       210                 215                 220
   Arg Glu Ile Ile Gly Trp Pro Gly Gly Ser Gly Asp Gly Ile Phe Ser
   225                 230                 235                 240
   Pro Gly Gly Ala Ile Ser Asn Met Tyr Ala Met Met Ile Ala Arg Phe
                   245                 250                 255
   Lys Met Phe Pro Glu Val Lys Glu Lys Gly Met Ala Ala Leu Pro Arg
               260                 265                 270
   Leu Ile Ala Phe Thr Ser Glu His Ser His Phe Ser Leu Lys Lys Gly
           275                 280                 285
   Ala Ala Ala Leu Gly Ile Gly Thr Asp Ser Val Ile Leu Ile Lys Cys
       290                 295                 300
   Asp Glu Arg Gly Lys Met Ile Pro Ser Asp Leu Glu Arg Arg Ile Leu
   305                 310                 315                 320
   Glu Ala Lys Gln Lys Gly Phe Val Pro Phe Leu Val Ser Ala Thr Ala
                   325                 330                 335
   Gly Thr Thr Val Tyr Gly Ala Phe Asp Pro Leu Leu Ala Val Ala Asp
               340                 345                 350
   Ile Cys Lys Lys Tyr Lys Ile Trp Met His Val Asp Ala Ala Trp Gly
           355                 360                 365
   Gly Gly Leu Leu Met Ser Arg Lys His Lys Trp Lys Leu Ser Gly Val
       370                 375                 380
   Glu Arg Ala Asn Ser Val Thr Trp Asn Pro His Lys Met Met Gly Val
   385                 390                 395                 400
   Pro Leu Gln Cys Ser Ala Leu Leu Val Arg Glu Glu Gly Leu Met Gln
                   405                 410                 415
   Asn Cys Asn Gln Met His Ala Ser Tyr Leu Phe Gln Gln Asp Lys His
               420                 425                 430
   Tyr Asp Leu Ser Tyr Asp Thr Gly Asp Lys Ala Leu Gln Cys Gly Arg
           435                 440                 445
   His Val Asp Val Phe Lys Leu Trp Leu Met Trp Arg Ala Lys Gly Thr
       450                 455                 460
   Thr Gly Phe Glu Ala His Val Asp Lys Cys Leu Glu Leu Ala Glu Tyr
   465                 470                 475                 480
   Leu Tyr Asn Ile Ile Lys Asn Arg Glu Gly Tyr Glu Met Val Phe Asp
                   485                 490                 495
   Gly Lys Pro Gln His Thr Asn Val Cys Phe Trp Tyr Ile Pro Pro Ser
               500                 505                 510
   Leu Arg Thr Leu Glu Asp Asn Glu Glu Arg Met Ser Arg Leu Ser Lys
           515                 520                 525
   Val Ala Pro Val Ile Lys Ala Arg Met Met Glu Tyr Gly Thr Thr Met
       530                 535                 540
   Val Ser Tyr Gln Pro Leu Gly Asp Lys Val Asn Phe Phe Arg Met Val
   545                 550                 555                 560
   Ile Ser Asn Pro Ala Ala Thr His Gln Asp Ile Asp Phe Leu Ile Glu
                   565                 570                 575
   Glu Ile Glu Arg Leu Gly Gln Asp Leu
           580                 585

## Description

The specification of the present invention relates to the use of recombinant DNA technology for the transformation of a host organism with glutamic acid decarboxylasa₆₅ (GAD₆₅) for the expression of GAD₆₅ polypeptides. Also described are methods of using GAD₆₅ polypeptides diagnostically and therapeutically in autoimmune disease.

Insulin-dependent diabetes mellitus (IDDM; type I diabetes) is one of the most common metabolic disorders. In the United States, IDDM affects approximately one in 300 to 400 people, and epidemiological studies suggest that its incidence is increasing. The disease results from the autoimmune destruction of the insulin-producing β-cells of the pancreas. More specifically, the preonset stage is characterized by "insulitis", in which lymphocytes infiltrate the pancreatic islets and selectively destroy the β-calls. The typical IDDM presentation of hyperglycemia appears only after at least 80% of the insulin-producing β-cells are lost. The remaining β-cells are destroyed during the next few years.

Although insulin therapy allows most IDDM patients to lead normal lives, this replacement is imperfect and does not completely restore metabolic homeostasis. Thus, severe complications which result in dysfunctions of the eye, kidney, heart and other organs are common in IDDM patients undergoing insulin therapy. Because of this, it is highly desirable to extend the latency period (e.g.,through administration of immunosuppressant drugs) between the start of β-cell destruction and the actual requirement of insulin replacement (i.e., when 80% of the β-cells are destroyed). Therefore, a diagnostic test which determines the beginning of β-cell destruction would allow the clinician to administer immunosuppressant drugs (Silverstein, *et al., New England Journal of Medicine*, 319:599-604, 1988) to extend this latency period and thus significantly delay the onset of insulin replacement side effects.

Many IDDM patients have sera which contain antibodies to a 64kD molecule (Baekkeskov, *et al., J.Clin.Invest.,* 79:926-934, 1987; Atkinson, *et al., Lancet,* 335:1357-1360, 1990), to islet cell cytoplasmic (ICA) molecules or islet cell surface (ICSA) molecules (Bottazzo, *et al, Lancet,* 1:668-672, 1980), or to insulin (Palmer, *et al., Science,* 222:1137-1139, 1983; Atkinson, *et al., Diabetes,* 35:894-898, 1986). Atkinson and coworkers (Atkinson, *et al., Lancet*, 335:1357-1360, 1990) have demonstrated that the presence of antibodies to the 64kD molecule in human sera appears to be the earliest and most reliable indicator that onset of IDDM symptoms will eventually occur.

Recently, Baekkeskov and coworkers established that the 64kD molecule and glutamic acid decarboxylase (GAD) have several antigenic epitopes in common and thus they may be identical or very similar molecules. Although this identification is an important finding, the use of this information as a diagnostic tool for predicting IDDM is quite cumbersome and limited unless knowledge of the molecular biology of GAD is known. Consequently, the cloning and subsequent production of large quantities of the 64kD molecule, or a GAD molecule which is antigenically substantially identical to the 64kD molecule, will allow the development of a diagnostic kit designed to predict IDDM. The present invention provides a means for accomplishing this result.

The present invention arose out of the discovery that recombinant DNA technology could be used to produce eukaryotic GAD₆₅ polypeptide and that GAD₆₅ polypeptide could be used in the diagnosis and therapy of patients with autoimmune disease. Particularly relevant is the use of cloned eukaryotic GAD₆₅ polypeptide in the diagnosis of patients having, or at risk of having, insulin-dependent diabetes mellitus (IDDM).

A major advantage of the present invention is that it provides the art with a ready source of eukaryotic GAD₆₅ polypeptide corresponding to that purified from natural sources, while avoiding the problems associated with the isolation of naturally occurring eukaryotic GAD₆₅ polypeptide when separating it from other eukaryotic non-GAD₆₅ polypeptides. This absence of other eukaryotic non-GAD₆₅ polypeptides is significant in that it allows the development of test systems which will only detect antibodies specifically reactive with GAD₆₅ polypeptides.

Another advantage of providing eukaryotic GAD₆₅ polypeptide in host cells is that by so doing, it is possible to obtain much larger quantities of the polypeptide than are currently practicably available from natural sources. As a consequence, not only is it possible to use the polypeptide of the invention to more accurately classify patients with such autoimmune diseases as IDDM, but it is also now possible to provide commercially useful quantities of GAD₆₅ polypeptide for use in diagnostic systems.
- FIGURE 1: Cloning strategy for obtaining GAD₆₅ and GAD₆₇ specific cDNA probes.
- FIGURE 2: DNA sequence and corresponding amino acid sequence for rat GAD₆₅.
- FIGURE 3: DNA sequence and corresponding amino acid sequence for human GAD₆₅.

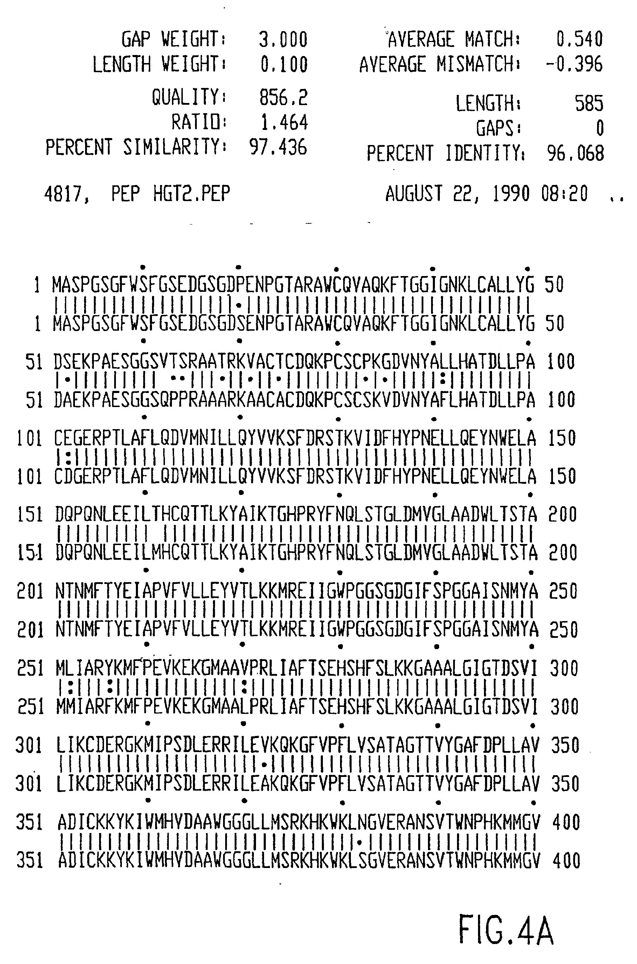

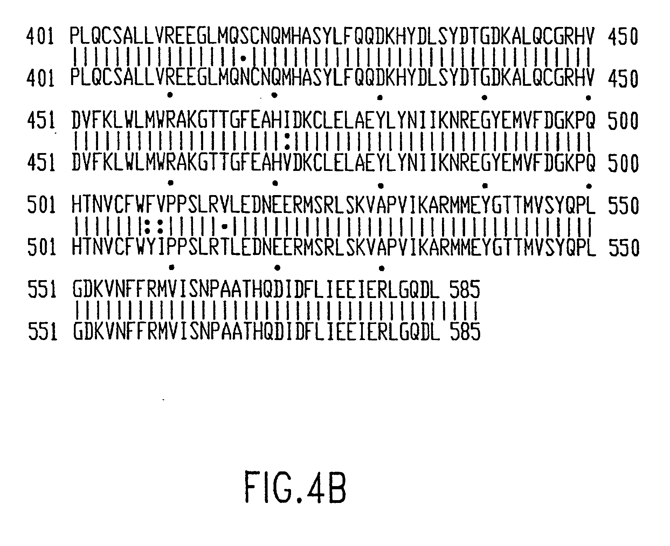

- FIGURE 4: Comparison of rat GAD₆₅ and human GAD₆₅ amino add sequences.
- FIGURE 5: GAD₆₅ and GAD₆₇ cDNAs hybridize to different size RNAs.
- FIGURE 6: Southern blots hybridized with cDNA probes specific for GAD₆₅ andGAD₆₇.
- FIGURE 7: Immunological identification of GAD₆₅ and GAD₆₇.

In particular, the present invention relates to various uses of a polypeptide, as specified in the appended claims.

The specification of the present invention relates to the manipulation of genetic materials by recombinant procedures which make possible the production of polypeptides possessing part of the primary structural conformation for one or more of the epitopes for binding autoantibodies to glutamic acid decarboxylase₆₅ (GAD₆₅). These polypeptides are highly useful for the immunological detection of autoantibodies reactive with them, since such autoantibodies are indicative of autoimmune diseases such as insulin dependent diabetes mellitus and "stiff man" syndrome. These polypeptides can also be used for purposes of screening drugs, such as those that alter GAD function, and for generation of polyclonal and monoclonal antibodies which, in turn, can be used diagnostically to detect GAD₆₅.

The development of specific DNA sequences encoding eukaryotic GAD₆₅ polypeptide for splicing into DNA vectors can be accomplished using a variety of techniques. For example, alternative methods which can be employed include (1) the isolation of a double stranded DNA sequence from the genomic DNA of the eukaryote; (2) the chemical manufacture of a DNA sequence to provide the necessary codons for the polypeptide of interest; and (3) the *in vitro* synthesis of a double stranded DNA sequence by reverse transcription of mRNA isolated from a eukaryotic donor cell. In the latter case, a double stranded DNA complement of mRNA is eventually formed which is generally referred to as cDNA.

The manufacture of DNA sequences is frequently the method of choice when the entire sequence of amino acid residues of the desired polypeptide product is known. When the entire sequence of amino acid residues of the desired polypeptide is not known, the direct manufacture of DNA sequences is not possible and the method of choice is the formation of cDNA sequences. Among the standard procedures for isolating cDNA sequences of interest is the formation of plasmid-carrying cDNA libraries which are derived from reverse transcription of mRNA which is abundant in donor cells that have a high level of genetic expression. When used in combination with polymerase chain reaction technology, even rare expression products can be cloned. In those cases where significant portions of the amino acid sequence of the polypeptide are known, the production of labeled single or double stranded DNA or RNA probe sequences duplicating a sequence putatively present in the target CDNA may be employed in DNA/DNA hybridization procedures which are carried out on cloned copies of the cDNA which have been denatured into a single stranded form (Jay, *et al., Nucleic Acid Research,* 11:2325, 1983).

Hybridization procedures are useful for the screening of recombinant clones by using labeled mixed synthetic oligonucleotide probes wherein each is potentially the complete complement of a specific DNA sequence in the hybridization sample which includes a heterogeneous mixture of denatured double stranded DNA. For such screening, hybridization is preferably performed on either single stranded DNA or denatured double stranded DNA. These procedures are particularly useful in the detection of cDNA clones derived from sources where an extremely low amount of mRNA sequences relating to the polypeptide of interest are present. In other words, by using stringent hybridization conditions directed toward avoidance of non-specific binding, it is possible, for example, to allow the autoradiographic visualization of a specific cDNA done by the hybridization of the target DNA to that single probe in the mixture which is its complete complement (Wallace, *et al., Nucleic Acid Research,* 9:879, 1981).

In addition, a GAD cDNA library can be screened by injecting the various cDNAs into oocytes, allowing sufficient time for expression of the cDNA gene products to occur, and testing for the presence of the desired cDNA expression product, for example, by using antibody specific for GAD₆₅ polypeptide or by using functional assays for GAD₆₅ enzymatic activity.

Alternatively, a cDNA library can be screened indirectly for GAD₆₅ peptides having at least one epitope using antibodies to GAD₆₅ (Chang and Gottlieb, *J.Neurosci.,* 8:2123, 1988). Such antibodies can be either polyclonally or monoclonally derived and used to detect expression product indicative of the presence of GAD₆₅ cDNA. Preferred are antibodies directed to an epitope found in the first 100 amino acids of the N-terminal portion of GAD₆₅.

Of the three above-noted methods for developing specific DNA sequences for use in recombinant procedures, the use of genomic DNA isolates is the least common. This is especially true when it is desirable to obtain the microbial expression of mammalian polypeptides because of the presence of introns

The specification of the present invention relates to novel polypeptides of GAD₆₅ which have part or all of the primary structural conformation, that is, a continuous sequence of amino acid residues, having at least one epitope for antibodies to GAD₆₅.

It is possible to use the polypeptide fragments of the invention rather than intact GAD₆₅ to detect autoantibodies to GAD₆₅. The term "polypeptide," as applied to GAD₆₅ polypeptide, denotes any sequence of amino acids having an epitope for autoantibodies to GAD₆₅, wherein the sequence of amino acids is encoded by all or part of the cDNA sequences of the invention.

The polypeptides resulting from microbial expression of the DNA sequences of the invention can be further characterized by their freedom from association with other eukaryotic polypeptides or other contaminants which might otherwise be associated with GAD₆₅ in its natural cellular environment or in such extracellular fluids as plasma or urine.

Studies by the present inventors unequivocally establish that GAD₆₅ and GAD₆₇ are encoded by distinct genes and are not produced, for example, by post-transcriptional or post-transiational modification of a common genomic sequence. Evidence proving that GAD₆₅ and GAD₆₇ are encoded by different genes include: (a) the largest contiguous sequence of exact identity between GAD₆₅ and GAD₆₇ cDNAs is only 17 nucleotides in length, (b) cDNAs from GAD₆₅ and GAD₆₇ do not cross hybridize with each other's or with each other's mRNA under low stringency conditions (2.0 x SSC, 0.01% SDS, 23°C), and (c) GAD₆₅ and GAD₆₇ cDNAs do not cross hybridize with isolated genomic dones encoding GAC₆₇ and GAD₆₅, respectively.

The term "host" is meant to include not only prokaryotes, but also such eukaryotes as yeast, filamentous fungi, as well as plant and animal cells which can replicate and express an intron-free DNA sequence of eukaryotic GAD₆₅. However, prokaryotes are preferred as the host organism.

The term "prokaryotes" is meant to include all bacteria which can be transformed or transfected with the gene for the expression of GAD₆₅. Prokaryotic hosts may include gram negative as well as gram positive bacteria such as, for example, *E. coli, S. typhimurium, Serratia marcascens and Bacillus subtills.*

A recombinant DNA molecule coding for the GAD₆₅ polypeptides can be used to transform or transfect the host using any of the techniques commonty known to those of ordinary skill in the art. Especially preferred is the use of a plasmid or a virus containing the GAD₆₅ coding sequence for purposes of prokaryotic transformation or transfection, respectively.

Methods for preparing fused, operably linked genes and expressing them in bacteria are well-known in the art (Maniatis, *et al., Molecular Cloning: A Laboratory Manual*, Cold Spring Harbor Laboratory, Cold Spring Harbor, NY, 1989). The genetic constructs and methods described therein can be utilized for expression of GAD₆₅ in prokaryotic hosts.

In general, expression vectors containing promotor sequences which facilitate the efficient transcription of the inserted eukaryotic genetic sequence are used in connection with the host The expression vector typically contains an origin of replication, a promoter, and a terminator, as well as specific genes which are capable of providing phenotypic selection of the transformed cells. The transformed prokaryotic hosts can be grown in fermentors and cultured according to techniques known in the art to achieve optimal cell growth. The polypeptides described herein can then be isolated from the grown medium, cellular lysates, or cellular membrane fractions.

The isolation and purification of the microbially expressed polypeptides described herein may be by any conventional means such as, for example, preparative chromatographic separations and immunological separations such as those involving the use of monoclonal or polyclonal antibody.

By having provided the sequence of amino acid residues of GAD₆₅, the specification of the present invention describes the manufacture of DNA sequences which code for the host expression of polypeptide analogs or derivatives of GAD₆₅ which differ from naturally-occurring forms in terms of the identity or location of one or more amino acid residues and which share some or all of the epitopes of naturally-occurring polypeptide forms.

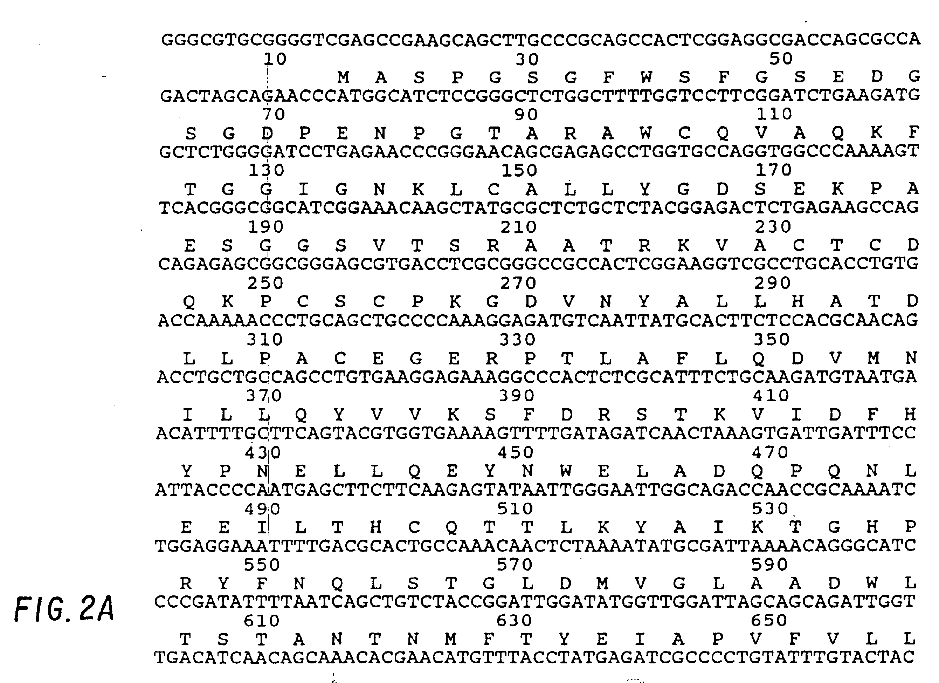

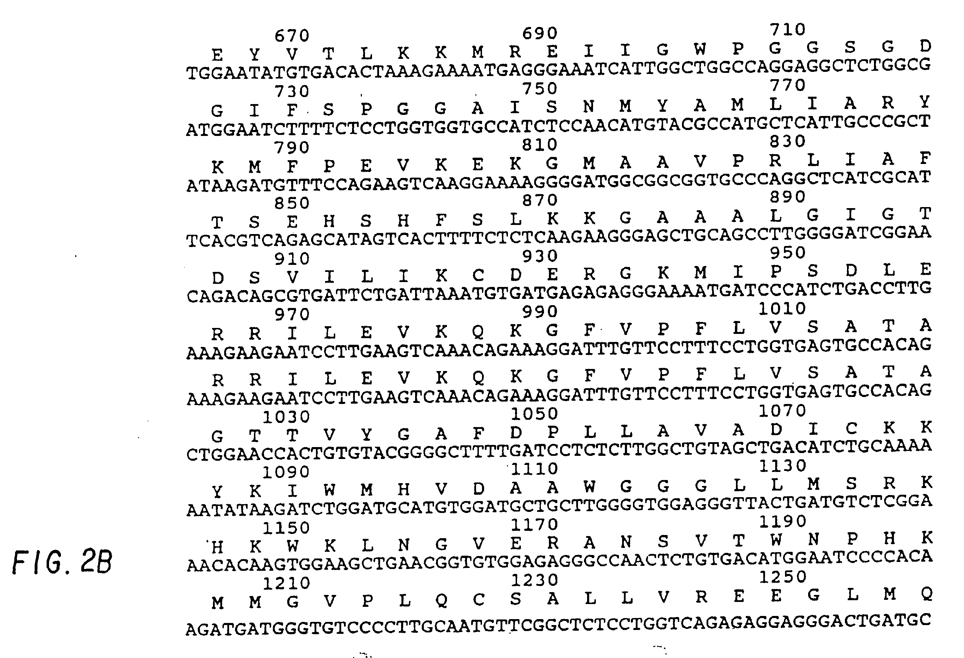

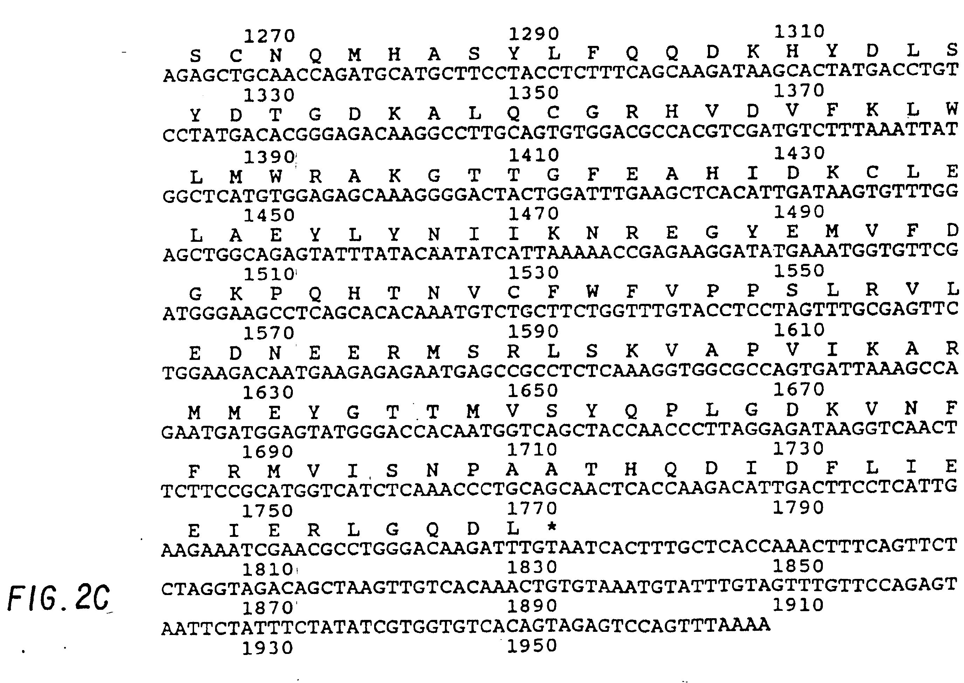

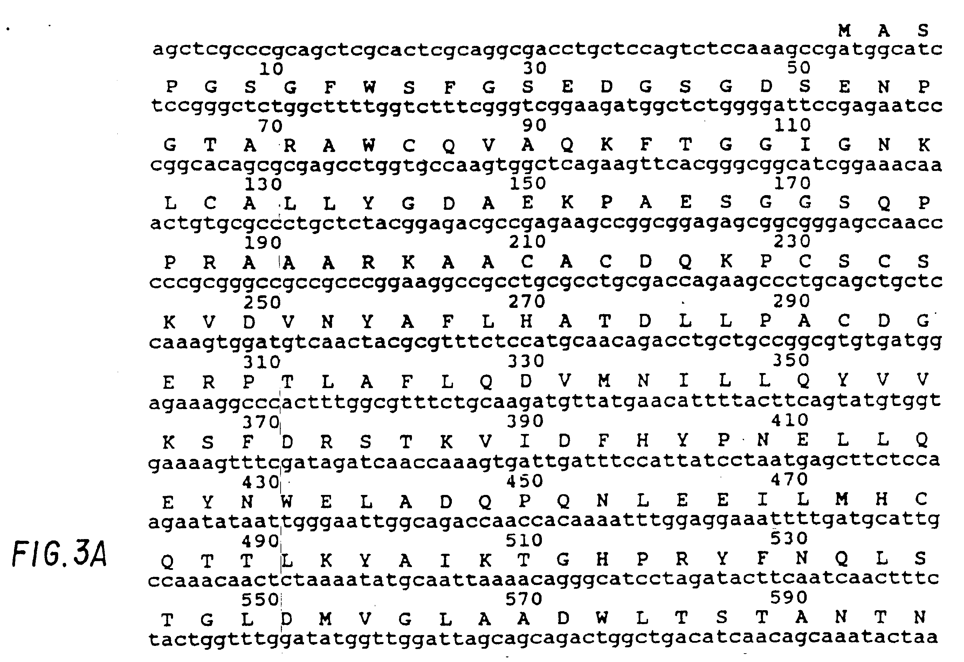

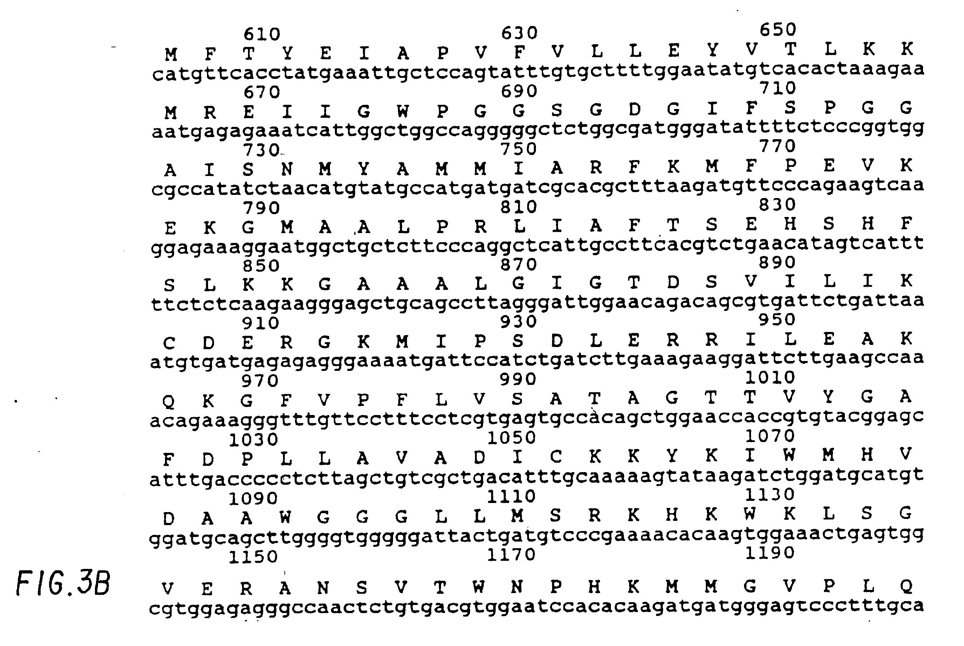

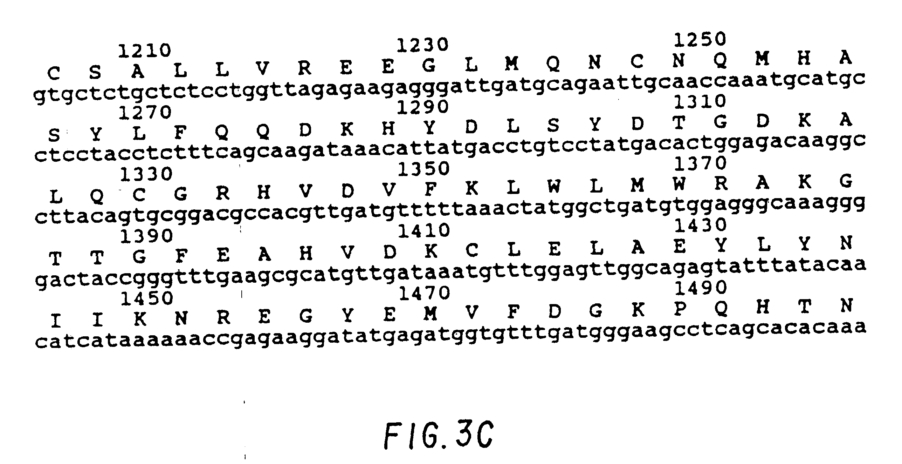

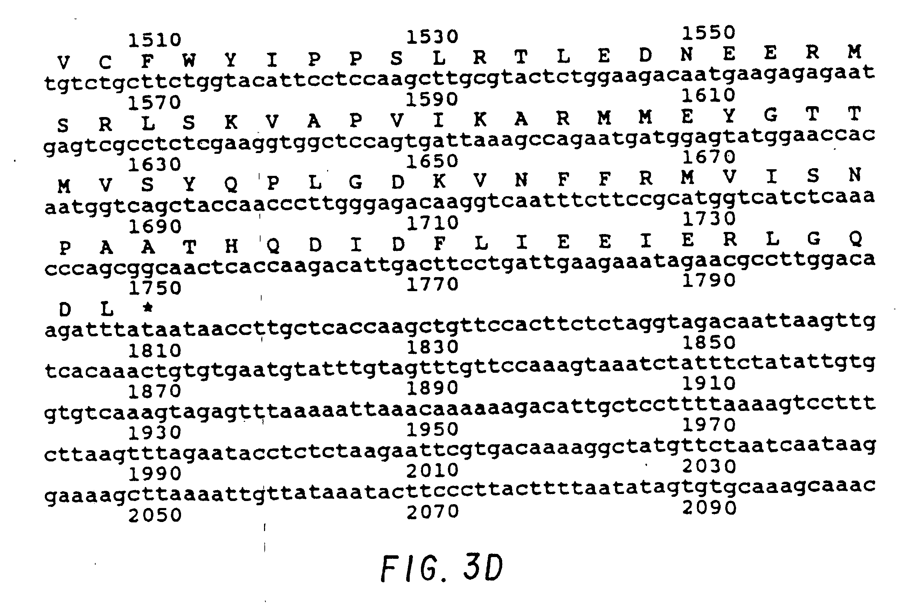

The novel DNA sequences indude all sequences useful in providing the expression in prokaryotic or eukaryotic host cells of polypeptides which have at least a part of the primary structural conformation for one or more epitopes capable of reacting with autoantibodies to GAD₆₅ which are comprehended by: (a) the DNA sequence as set forth in Figures 2 or 3 or their complementary strands; (b) DNA sequences which hybridize to DNA sequences defined in (a) or fragments thereof; and (c) DNA sequences which, but for the degeneracy of the genetic code, would hybridize to DNA sequences defined in (a) and (b) above. Specifically comprehended in (b) are genomic DNA sequences which encode allelic variant forms of GAD₆₅. Part (c) specifically comprehends the manufacture of DNA sequences which encode GAD₆₅, GAD₆₅ fragments, and GAD₆₅ analogs wherein the DNA sequences thereof may incorporate codons which facilitate translation of mRNA in non-vertebrate hosts.

Since the cDNA sequence described herein encodes essentially the entire human or rat GAD₆₅ molecule, it is now a matter of routine to prepare, subclone, and express smaller polypeptide fragments of cDNA from this or a corresponding cDNA sequence which would encode as few as one epitope for autoantibodies to human or rat GAD₆₅. The presence of such an epitope on a cloned polypeptide can then be confirmed using, for example, sera from a patient with autoantibodies to GAD₆₅. An example of such a smaller peptide is the first approximately 100 amino acids from the N-terminus of GAD₆₅ (shown in Figure 3). This amino acid sequence is essentially absent from GAD₆₇.

The GAD₆₅ described herein is particularly suited for use in immunoassays in which it can be utilized in liquid phase or bound to a solid phase carrier. In addition, GAD₆₅ used in these assays can be detectably labeled in various ways.

Examples of immunoassays which can utilize the GAD₆₅ are competitive and non-competitive immunoassays in either a direct or indirect format. Examples of such immunoassays are the radioimmunoassay (RIA), the sandwich (immunometric assay) and the Western blot assay. Detection of antibodies which bind to the GAD₆₅ can be done utilizing immunoassays which run in either the forward, reverse, or simultaneous modes, including immunohistochemical assays on physiological samples. The concentration of GAD₆₅ which is used will vary depending on the type of immunoassay and nature of the detectable label which is used. However, regardless of the type of immunoassay which is used, the concentration of GAD₆₅ utilized can be readily determined by one of ordinary skill in the art using routine experimentation.

The GAD₆₅ can be bound to many different carriers and used to detect the presence of antibody specifically reactive with the polypeptide. Examples of well-known carriers indude glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for purposes of the invention. Those skilled in the art will know of other suitable carriers for binding GAD₆₅, or will be able to ascertain such, using routine experimentation.

There are many different labels and methods of labeling known to those of ordinary skill in the art. Examples of the types of labels which can be used in the present invention include enzymes, radioisotopes, colloidal metals, fluorescent compounds, chemiluminescent compounds, and bioluminescent compounds.

Alternatively, the polypeptide described herein can be used to detect antibodies to GAD₆₅ by measuring GAD enzymatic activity. For example, GAD₆₅ and a specimen suspected of having antibodies to GAD₆₅ can be incubated for a period of time and under conditions sufficient to allow binding to occur between GAD₆₅ and the antibodies. The reaction product is precipitated and then tested for GAD enzymatic activity.

For purposes of the invention, the antibody which binds to GAD₆₅ may be present in various biological fluids and tissues. Any sample containing a detectable amount of antibodies to GAD₆₅ can be used. Normally, a sample is a liquid such as urine, saliva, cerebrospinal fluid, blood, serum and the like, or a solid or semi-solid such as tissue, feces and the like.

The materials for use in the assay of the invention are ideally suited for the preparation of a kit. Such a kit may comprise a carrier means being compartmentalized to receive in close confinement one or more container means such as vials, tubes and the like, each of the container means comprising one of the separate elements to be used in the method. For example, one of the container means may comprise GAD₆₅ bound to a carrier. A second container may comprise soluble, datectably-labeled second antibody, in lyophilized form or in solution.

In addition, the carrier means may also contain a plurality of containers each of which comprises different, predetermined amounts of GAD₆₅. These latter containers can then be used to prepare a standard curve into which can be interpolated the results obtained from the sample containing the unknown amount of autoantibodies to GAD₆₅.

In using the kit all the user has to do is add, to a container, a premeasured amount of a sample containing a measurable, yet unknown amount of autoantibodies to GAD₆₅ to be detected, a premeasured amount of carrier-bound GAD₆₅ present in the first container, and a premeasured amount of the detectably labeled second antibody present in the second container. Alternatively, the non-detectably labeled GAD₆₅ can be provided attached to the container to which the sample and the detectably labeled second antibody are added. After an appropriate time for incubation, an immune complex is formed and is separated from the supernatant fluid, and the immune complex or the supernatant fluid are detected, as by radioactive counting or addition of an enzyme substrate, and color development.

The term "ameliorate" denotes a lessening of the detrimental effect of the autoimmune response in the patient receiving therapy. The term "therapeutically effective" means that the amount of GAD₆₅ polypeptide used is of sufficient quantity to ameliorate the cause of disease due to the autoimmune response.

The recombinant GAD₆₅ polypeptides can also be used therapeutically in patients having an autoimmune response to GAD₆₅ Such therapy can be accomplished by, for example, the administration of recombinant GAD₆₅ polypeptide. Such administration can utilize unlabeled as well as labeled GAD₆₅ polypeptide. When unlabeled GAD₆₅ polypeptide is utilized advantageously, it would be in a form wherein, for example, the GAD₆₅ polypeptides are in fragments which are too small to stimulate an immune response, but large enough to bind, or block, the continuance of the autoimmune response. For example, GAD₆₅ could be digested enzymatically into epitope-sized peptides (typically 5-12 amino acids in length) and thereby bind to Fab binding portions present in the body fluids, or on the surface of immune cells, of the patient with autoimmune disease.

Alternatively, the recombinant GAD₆₅ polypeptides could be administered labeled with a therapeutic agent. These agents can be coupled either directly or indirectly to the GAD₆₅ polypeptides. One example of indirect coupling is by use of a spacer moiety. These spacer moieties, in turn, can be either insoluble or soluble (Diener, *et al., Science*, 231:148, 1986) and can be selected to enable drug release from the GAD₆₅ polypeptide at the target site. Examples of therapeutic agents which can be coupled to the GAD₆₅ polypeptides of the invention for immunotherapy are drugs, radioisotopes, lectins, and toxins.

The drugs with which can be conjugated to the GAD₆₅ polypeptides of the invention include compounds which are classically referred to as drugs such as mitomycin C, daunorubicin, and vinblastine.

In using radioisotopically conjugated GAD₆₅ polypeptides of the invention for immunotherapy, certain isotopes may be more preferable than others depending on such factors as leukocyte distribution as well as stability and emission. Depending on the autoimmune response, some emitters may be preferable to others. In general, a and β particle-emitting radioisotopes are preferred in immunotherapy. Preferred are short range, high energy a emitters such as ²¹²Bi. Examples of radioisotopes which can be bound to the GAD₆₅ polypeptides of the invention for therapeutic purposes are ¹²⁵I, ¹³¹I, ⁹⁰Y, ⁵⁷Cu, ²¹²Bi, ²¹¹At, ²¹²Pb, ⁴⁷Sc, ¹⁰⁹Pd and ¹⁸⁸Re.

Lectins are proteins, usually isolated from plant material, which bind to specific sugar moieties. Many lectins are also able to agglutinate cells and stimulate lymphocytes. However, ricin is a toxic lectin which has been used immunotherapeutically. This is accomplished by binding the α-peptide chain of ricin, which is responsible for toxicity, to the antibody molecule to enable site specific delivery of the toxic effect.

Toxins are poisonous substances produced by plants, animals, or microorganisms that in sufficient dose, are often lethal. Diphtheria toxin is a substance produced by *Corynebacterium diphtheria* which can be used therapeutically. This toxin consists of an a and β subunit which under proper conditions can be separated. The toxic A component can be bound to GAD₆₅ polypeptide and used for site specific delivery to a leukocyte expressing a receptor for GAD₆₅ polypeptide.

Other therapeutic agents which can be coupled to the GAD₆₅ polypeptides as well as *ex vivo* and *in vivo* therapeutic protocols, are known, or can be easily ascertained, by those of ordinary skill in the art.

The specification of the present invention also mentions that the polypeptide described herein can be administered therapeutically to ameliorate the disease process in patients having, or at risk of having, this disease. The conventional single-letter code used to represent the various amino acids relates as follows:

| | | | |
|---|---|---|---|
| Phe: F | Leu: L | Ile: I | Met: M |
| Val: V | Ser: S | Pro: P | Thr: T |
| Ala: A | Tyr: Y | His: H | Gln: Q |
| Asn: N | Lys: K | Asp: D | Glu: E |
| Cys: C | Trp: W | Arg: R | Gly: G |

The polypeptide-sequence of the polypeptide described herein was identified by comparing the amino acid sequences of human GAD₆₅, human GAD₆₇, and the P2-C protein of the picomavirus, coxsackie virus. The P2-C polynucleotide plays a role in the virus membrane bound replication complex. These analyses established the presence of an extensive sequence similarity between both GAD₆₅ molecules and the coxsackie virus. A core polypeptide of six contiguous amino acid residues of the GAD₆₅ and P2-C polypeptide are identical in amino acid sequence. Indeed, of the 24 amino acids in the polypeptide, 19 are identical or conserved. In addition, there also exists a high charge density and the presence of a proline residue which would render this region highly antigenic (see Table 1).

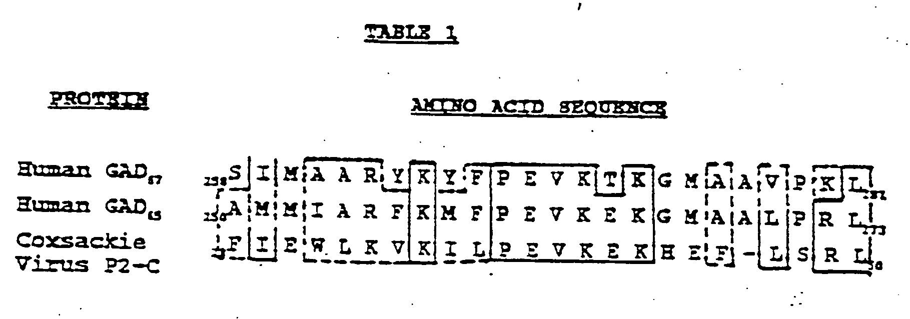

In Table 1, the solid line encloses identical amino acids whereas the dashed line encloses amino acid residues with similar charge, polarity, or hydrophobicity.

The discovery of this common polypeptide region supports an etiologic role for molecular mimicry in the precipitation of diabetes. Thus, where a patient genetically susceptible to IDDM is infected by a coxsackie virus, the immune response to the coxsackie virus polypeptide results in a cross reactive immune response to the similar GAD sequence in the patients β-cells. The immunological response is maintained by the antigenically similar GAD polypeptides resulting in the eventual destruction of the β-cells and the subsequent presentation of IDDM.

At present, it is believed that the elimination of pancreatic β-cells is mediated by a cellular autoimmune response. Consequently, a polypeptide as described herein should have the capability of blocking such cellular autoimmune response. Because of the complexity of autoimmune disease, it is possible to envision numerous possible therapeutic modalities which would allow the polypeptides of the invention to be used to ameliorate such diseases. Thus, it may be possible to utilize the polypeptides to block recognition by a specific T call receptor (TCR) or an MHC receptor presenting an autoimmune antigen on the surface of an antigen presenting cell (APC). The inhibition of such recognition might occur, for example, by providing the patient with the polypeptide of the invention which, in turn, could displace the autoimmune antigen being presented in the antigen-cieft of the MHC receptor or, possibly, by direct interaction with the appropriate TCR on the surface of a T-helper cell. This latter therapeutic approach of direct interaction with the TCR could be achieved through induction of high-zone tolerance by use of high concentrations of soluble polypeptide

Alternatively, the polypeptides described herein could be used to stimulate a T-suppressor cell population in order to restore self-recognition and, thereby, ameliorate the autoimmune disease. Stimulation of T-suppressor cell populations could be achieved, for example, by use of a bi-specific antibody having one variable region specific for an epitope present on the autoimmune antigen residing in the cleft of the MHCII receptor and, a second variable region specific for an epitope present on the CD8⁺ receptor. The production of antibody specific for the polypeptide described herein is a matter of routine to those of skill in the art, as is the preparation of bi-specific antibodies having specificity for 2 or more epitopes.

Polypeptide analogs of the polypeptide described herein may be designed which will compete for recognition of setf-antigens at the level of antigen presentation. Since MHC molecules contain a single peptide binding site, it is possible to design polypeptides which will bind with high affinity to disease-associated MHC molecules, but will not activate disease-causing T-heiper cells. Such polypeptides act as antagonists for self-antigen recognition. Precedent for such an approach arises from observation that a mouse lysozyme polypeptide, itself non-immunogenic, can compete for MHC binding with an immunogenic polypeptide from hen-egg white lysozyme and thereby reduce T cell activation by that polypeptide (Adarini, *et al., Nature,* 334:623-625, 1988). Similarly, such a therapeutic approach for screening effective polypeptide analogs has been utilized in such autoimmune diseases as experimental autoimmune encephalomyelitis (EAE) (Wraith, *et al., Cell,* 59:248, 1989; Urban, *et al., Cell*, 59:257, 1989).

The single-letter symbols used to represent the amino acid residues in the polypeptides of the present invention are those symbols commonly used in the art. The term "analog" refers to any polypeptide having a substantially identical amino acid sequence to a polypeptide provided herein and in which one or more amino acids have been substituted with chemically similar amino acids. For example, one polar amino acid, such as glycine or serine, may be substituted for another polar amino acid; or one acidic amino acid, such as aspartic acid may be substituted for another acidic amino acid, such as glutamic add; or a basic amino acid, such as lysine, arginine, or histidine may be substituted for another basic amino acid; or a non-polar amino add such as alanine, leucine, or isoleucine may be substituted for another non-polar amino acid.

The term "analog" also means any polypeptide which has one or more amino acids deleted from or added to a polypeptide of the present invention, but which still retains a substantial amino acid sequence homology to such peptide. A substantial sequence homology is any homology greater than 50%. The term "fragment" means any shorter version of the polypeptides identified herein having at least 6 amino acid residues, wherein the fragment is capable of stimulating proliferation of islet infiltrating T lymphocytes (HTLs), or is a fragment capable of inhibiting the stimulation of such calls by a stimulating polypeptide fragment.

The term "chemical derivative" means any polypeptide derived from a polypeptide as described herein and in which one or more amino acids have been chemically derivatized by reaction of the functional side groups of amino acid residues present in the polypeptide. Thus, a "chemical derivative" is a polypeptide that is derived from the sequences or polypeptides identified herein by one or more chemical steps. Such derivatized molecules include, for example, those molecules in which free amino groups have been derivatized to form amine hydrochlorides, P-toluene sulfoamides, benzoxycarboamides, T-butyloxycarboamides, thiourethane-type derivatives, trifluoroacetylamides, chloroacetylamides, or formamides. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-im-benzyihistidine. Also included as chemical derivatives are those polypeptides which contain one or more naturally occurring amino adds derivatives of the 20 standard amino adds. For example, 4-hydroxyproline may be substituted for proline; 5-hydroxylysine may be substituted for lysine; 3-methylhistidine may be substituted for histidine; homoserine may be substituted for serine, and ornithine may be substituted for lysine.

It should be understood that the present invention is not limited to the illustrative polypeptides depicted in Table 1, instead, a polypeptide falling within the scope of this invention may extend outside of, or comprise less than, the region between amino acid 28 and amino acid 50 of coxsackie virus P2-C, or between amino acid 250 and amino add 273 of GAD₆₅, or between amino add 258 and amino add 281 of GAD₆₇, as long as a substantial part of a given polypeptide is characterized by an amino acid sequence from that region, or segments or combinations thereof, and the polypeptide demonstrates the desired immunological or biological activity against autoimmune disease. In addition, polypeptides according to this invention indude those having amino acid sequences which are longer or shorter in length than those of the polypeptides illustrated in Table 1, or which comprise segments or combinations thereof, as long as such polypeptides consist substantially of the region between the amino acids illustrated in Table 1 and demonstrate immunological or biological activity. Furthermore, polypeptides according to this invention indude those characterized by a sequence of amino acids which is longer or shorter than that of the GAD₆₅ polypeptide of Table 1, or which comprise segments of that polypeptide and which display immunological or biological activity against the autoimmune disease. All polypeptides of the invention should not stimulate or enhance the autoimmune disease.

Accordingly, it should be understood that the specific selection of any one polypeptide within the polypeptides of the invention does not involve undue experimentation. Such a selection may be carried out by taking a number of polypeptides and testing them for their immunological and biological activity in ameliorating the autoimmune disease. The NOD mouse represents an excellent and well characterized model for screening polypeptides of the invention capable of ameliorating diabetes.

The polypeptides described herein may be prepared by recombinant techniques or by conventional synthesis using known polypeptide synthetic methods, including synthesis on a solid support. An example of a suitable solid phase synthetic technique is that described by Merriweather (*J.Am. Chem.Soc.,* 85:21 49, 1963). Other polypeptide synthetic techniques may be found, for example, in Bodanszky, *et al., Peptide Synthesis,* John Wiley & Sons, 2d-ed., 1976, as well as other references known to those skilled in the art . A summary of polypeptide synthesis techniques can be found in Stewart, *et al., Solid Phase Peptide Synthesis*, Pierce Chemical Company, Inc., Rockford, III., 1984. The synthesis of polypeptides by solution methods may also be used, for example, as described in *The Proteins*, Vol. II, 3d ed., Neurath, *et al., eds.*, 105, Academic Press, New York, NY, 1976. Appropriate protective groups for use in such synthesis can be found in the above references as well as in J. McOmie, *Protective Groups in Organic Chemistry*, Plenum Press, New York, NY, 1973.

The polypeptides described herein may also be prepared in an appropriate host transformed with DNA sequences that code for the desired polypeptide. For example, a polypeptide may be prepared by the fermentation of appropriate hosts that have been transformed with and which express a DNA sequence encoding the polypeptide. Alternatively, a DNA sequence encoding several of the polypeptides may be linked together and those sequences may then be used to transform an appropriate host to permit the expression of polypeptides involved in the autoimmune disease.

The dosage ranges for the administration of the GAD₆₅ polypeptides described herein are those large enough to produce the desired effect in which the symptoms or cellular destruction of the autoimmune response are ameliorated. The dosage should not be so large as to cause adverse side effects, such as unwanted cross-reactions, anaphylactic reactions, and the like. Generally, the dosage will vary with the age, condition, sex, and extent of the disease in the patient and can be determined by one of skill in the art. The dosage can be adjusted by the individual physician in the event of any counterindications. Dosage can vary from about 0.1mg/m² to about 2000mg/m². preferably about 0.1mg/m² to about 500mg/m²/dose, in one or more dose administrations daily, for one or several days.

The GAD₆₅ polypeptides can be administered parenterally by injection or by gradual perfusion over time. The GAD₆₅ polypeptides of the invention can be administered intravenously, intraperitoneally, intra-muscularly, subcutaneously, intracavity, or transdermally.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. intravenous vehicles include fluid and nutrient replenishers, electrolyte replenishers (such as those based on Ringer's dextrose), and the like. Preservatives and other additives may also be present such as, for example, antimicrobials, antl-oxidants, chelating agents, and inert gases and the like.

The specification of the invention also refers to a method for preparing a medicament or pharmaceutical composition comprising the GAD₆₅ polypeptides, the medicament being used for therapy of autoimmune response to GAD₆₅.

The above disclosure generally describes the present invention. A more complete understanding can be obtained by reference to the following specific examples which are provided herein for purposes of illustration only and are not intended to limit the scope of the invention.

### EXAMPLE 1

### CLONING AND EXPRESSION OF GAD₆₅

### A. RECOMBINANT DNA PROCEDURES

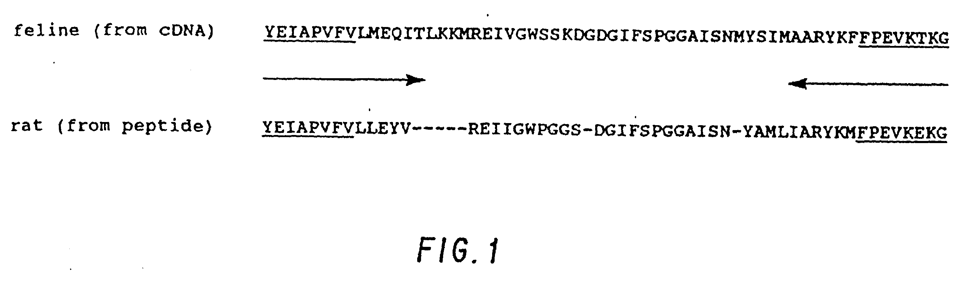

In order to obtain cDNA probes specific for GAD₆₅ and GAD₆₇, total RNA was extracted from adult rat brain by guanidine isothiocyanate-cesium gradient using the method of Chirgwin, *et al.* (Biochemistry, 18:5294, 1979). Poly (A) RNA was purified on ofigo dT cellulose, using the protocol by Bethesda Research Laboratories (BRL). First strand synthesis was performed by using MMLV-reverse transcriptase (BRL), with conditions suggested, except that poly d(N₈)-mers (Pharmacia) were used as primers. This cDNA-RNA mixture was heat inactivated at 65°C for 15 min and stored at -20°C. For PCR, 1/50 of the sample was added to the 100µl reaction. Degenerate oligonucleotides were synthesized (Applied Biosystems) to encode the underlined common amino acid sequences of feline (from cDNA) (Kobayashi, *et al., J.Neurosci.,* 7:2768, 1987) and rat (from peptides) (Chang and Gottlieb, *J.Neurosci.,* 8:2123, 1988) GAD (Figure 1). The 5'-end sequence of each degenerate oligonucleotide contained one strand of the DNA sequence recognized by either SstI and HindIII (5' oligo) or SstI and SstlI (3'-end oligo). These primers were used for selective amplification by polymerase chain reaction of the generated cDNA template as described by Gould, *et al. (Proc.Natl.Acad.Sci.,USA,* 86:1934, 1989). PCR products were subcloned into HindIII/SstI double digested Bluescript SK vector (Stratagene), transformed into DH5 (BRL), and plated by standard methods (Maniatis, *et al., Molecular Cloning: A Laboratory Manual,* Cold Spring Harbor Laboratory, Cold Spring Harbor, NY, 1989).

Colony hybridization was done with an 5'-³²P end labeled oligonucleotide specific to feline GAD₆₇ (Kobayashi, *et al., J.Neurosci.,* 7:2768, 1987). End labeling of oligonucleotide, hybridization conditions, and washing conditions were done as described (Wallace, *et al.,* in *Guide to Molecular Cloning Techniques;* Berger, *et al.,* Eds. in *Methods of Enzymology; Abelson, et al.,* Eds. *Academic Press, Inc.,* San Diego, 432-442, 1987), except that the nitrocellulose filters were washed at 50°C for 15 min. Colonies which were positive and negative in the hybridization were individually picked and grown overnight in Terrific Broth (Tartof, *et al., Focus*, 9:12, 1987). DNA was isolated using a boiling method (Maniatis, *et al., Molecular Cloning: A Laboratory Manual*, Cold Spring Harbor Laboratory, Cold Spring Harbor, NY, 1989) and templates were denatured by 0.2N NaOH and purified by Sephacryl S400 spun columns (Pharmacia). Sequencing of denatured double stranded template was by the chain-termination method (Sanger, *et al., Proc.Natl.Acad.Sci.,USA,* 74:5463, 1977) using the T7-sequencing kit (Pharmacia).

As shown in Figure 1, PCR-generated rat GAD₆₅ and GAD₆₇ cDNAs were used as probes to screen a lambda ZAP (Stratagene) rat hippocampus library provided by S. Heinemann (Salk Institute) by standard techniques (Maniatis, *et al., Molecular Cloning: A Laboratory Manual*, Cold Spring Harbor Laboratory, Cold Spring Harbor, NY, 1989). A 2400 nucleotide GAD₆₅ cDNA (the largest done) was isolated and subcloned by "zapping" as described by Stratagene. When a rat GAD₆₇ cDNA was obtained which was smaller than a 3.2kb rat GAD₆₇ cDNA done already on hand, the larger cDNA was sequenced. Exo III deletions (Henikoff, *Gene*, 28:351, 1984) were made in both directions for GAD₆₅ and GAD₆₇ and templates were prepared and sequenced as described above. Anchored PCR (Frohman, *et, al.*, *Proc.Natl.Acad.Sci.,USA,* 85:8998, 1988) was done to clone the remaining 5'-ends of GAD₆₅ and GAD₆₇ mRNAs which were not represented in the original cDNA clones isolated in the library screening. Sequencing of these clones revealed that neither GAD₆₅ nor GAD₆₇ mRNAs contained any further initiation codons (AUGs) in frame with the previously designated initiation codons of the original cDNA clones.

### EXAMPLE 2

### CHARACTERIZATION OF CLONED GAD₆₅

### A. NORTHERN BLOT HYBRIDIZATION

Two PCR-derived cDNA probes were hybridized to Northern blots containing rat brain RNA in order to determine whether the GAD₆₇ and GAD₆₅ cDNAs were derived from two different mRNAs. RNA was extracted as described in Example 1. Poly (A) RNA was separated by electrophoresis in formaldehyde and transferred onto Biotrans (ICN) membranes, and hybridization was performed as described by Well, *et al.* (*J.Neurosci.,* 16:311, 1986), except that 100µl/ml of poly (A) was added. Probes were labeled to approximately 10⁹ dpm/µg by the oligolabeling procedure of Feinberg and Vogelstein (*Anal.Biochem.,* 132:6. 1983). Identical results were subsequently obtained with full-tength clones of GAD₆₅, and GAD₆₇ cDNAs.

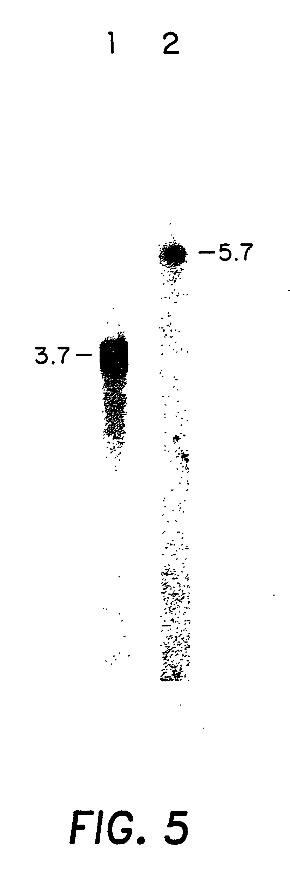

As shown in Figure 5, lanes 1 and 2 contain 1 µg of poly (A) selected RNA extracted from rat cerebellum. Lane 1 was hybridized to a cDNA probe for the rat cognate of feline GAD₆₇ (Kobayashi*, et al., J.Neurosci*., 7:2768, 1987) and lane 2 with a cDNA probe for the rat peptide sequence (which corresponds to GAD₆₅).

The cDNA probe for the rat peptide sequence hybridized to a 5.7kb RNA, while the cDNA probe for the rat cognate of our feline cDNA, hybridized to a 3.7kb RNA. This demonstrates that GAD₆₅ and GAD₆₇ are not derived from the same mRNA.

### B. GENOMIC HYBRIDIZATION OF GAD₆₇AND GAD₆₅

In order to investigate the possibility that GAD₆₇ and GAD₆₅ arise from separate genes, cDNAs of both GAD₆₇ and GAD₆₅ were hybridized to DNA blots containing genomic DNA.

For Southern blots, genomic DNA was extracted from rat liver as described (Kaiser, *et al.,* in *DNA Cloning*, vol.I, A Practical Approach, D.M. Glover ed., IRL Press, Oxford, _38-40, 1985). DNA (10µg/sample) was digested to completion with EcoRI and HindIII using conditions recommended by the suppliers (BRL, Gaithersburg, MD). DNA fragments were separated by electrophoresis at 1.5v/cm for 16 hrs in 0.8% agarose. The DNA was then transferred to Zeta-Probe membranes (Bio-Rad), hybridized, and washed, as described by Gatti, *et al*. (*Biotechniques,* 2:148, 1984), except that 5µg/ml Carnation dried milk was substituted for Denhardt's solution. Probes for Southern blots were labeled as described in Example 1, above.

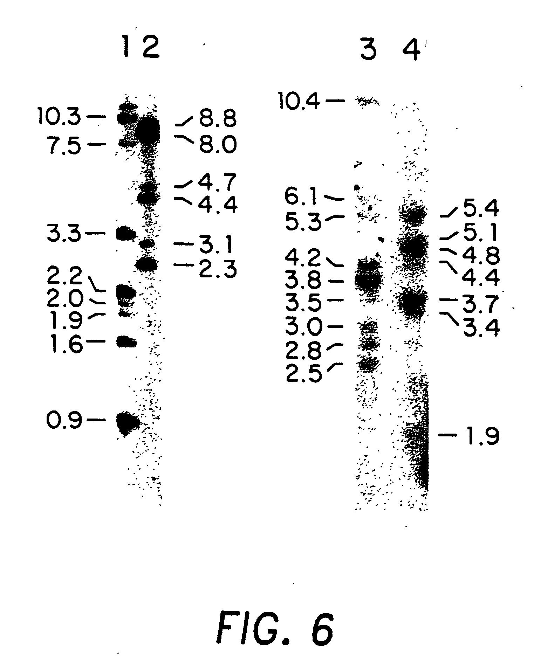

As shown in Figure 6, genomic DNA digested with HindIII and EcoRI are in lanes 1 and 3 and lanes 2 and 4, respectively. GAD₆₇ cDNA was hybridized to lanes 1 and 2, whereas GAD₆₅ cDNA was hybridized to lanes 3 and 4. Numbers along the side of the gel are the DNA fragment sizes in kilobases.

This data shows that the two cDNAs hybridize to genomic fragments of different sizes. In addition, the greatest contiguous stretch of identical nucleotide sequence of GAD₆₅ and GAD₆₇ cDNAs is only 17 nucleotide bases in length. Thus, GAD₆₇ and GAD₆₅ are encoded by two distinct genes.

### C. ENZYMATIC COMPARISON OF GAD₆₇ AND GAD₆₅

Studies were done comparing the effect of PLP on the activity of GAD₆₇ and GAD₆₅. In so doing, both cDNAs were subdoned into vectors that allowed their expression in bacteria (Studier, *et al., J.Mol.Biol.*, 189:113, 1986). Overexpression of "fusionless" GAD₆₅ and GAD₆₇ was accomplished by subcloning GAD₆₅-cDNA into the NcoI site of pET-8c and GA₆₇ cDNA into the Nhel site of pET-5c vectors (Studier, *et al., J.Mol.Biol.*, 189:113, 1986).

To obtain compatible sticky ends for correct in-frame subcloning of both cDNAs, selective amplification was performed by PCR using conditions suggested by United States Biochemical (USB), with 200µM dNTPs and 1.5mM MgCl₂ in the mixture and annealing at 55°C with 20 cycles to decrease infidelity of AmpliTAQ (USB). Primers specific for GAD₆₅ and GAD₆₇ contained one DNA strand of the NcoI and SpeI recognition sites, respectively. Since there is a NheI restriction site within the coding region of GAD₆₇, SpeI (which is compatible with NheI) was used.

PCR products were subdoned into their respective pET vectors, transformed into DH5 and plated as described (Maniatis, *et al.*, *Molecular Cloning: A Laboratory Manual*, Cold Spring Harbor Laboratory, Cold Spring Harbor, NY, 1989). Colonies were picked and grown overnight in LB broth with 50µg/ml ampicillin. Subclones with correct orientation were transformed into BL21(DE3) strain (Studier, *et al., J.Mol.Biol.,* 189:113, 1986) for overexpression. As a negative control, the pET-8C vector with no insert was transformed and subsequently induced. Single colonies were picked, grown, induced by 1 mM isopropyl-B-D-thiogaiacto-pyranoside (IPTG), and analyzed on SDS-PAGE gels as described (Sambrook *et al., Molecular Cloning a Laboratory Manual*, Cold Spring Harbor Laboratory Press, Cold Spring Harbor, 17.15-17.16, 1989).

To measure GAD activity, we induced 10ml cultures of bacteria at OD₆₀₀-0.5 with 1 mM IPTG. Two hours after induction, bacteria was spun down and resuspended and sonicated in 1 ml of homogenizing buffer (1mM phenylmethylsulfonyl fluoride (PMSF), 1mM 2-aminoethylisothiouronium bromide (AET), and 60mM potassium phosphate, pH 7.1). After sonication, call debris was removed by centrifugation and protein concentration was measured (Bradford, *Anal.Biochem.,* 72:248, 1986) in the supernatant (supernatant was stored in aliquots at -70°C). Brain homogenates were prepared as described (Legay, *et al., J.Neurochem*., 46:1478, 1986). GAD activity was measured as described (Krieger, *et al., J.Neurochem* 33:299, 1984) with 0.2mM PLP present or absent and 20µl of brain homogenate or bacterial lysate in the incubation mixture. Production of ¹⁴CO₂ In bacterial lysates was linear with respect to time of incubation and protein concentration.

**TABLE 2**

| **Source** | **GAD Specific Activity^{a}** | | **Fold Increase in Induction** |
|---|---|---|---|
| | **-PLP** | **+PLP** | |
| BL21(DE3) + pET-8c | 12 ± 0.4 | 9 ± 1 | -- |
| BL21(DE3) + pET-GAD₆₅ | 115 ± 3 | 773 ± 61 | 6.7 |
| BL21(DE3) + pET-GAD₆₇ | 160 ± 2 | 389 ± 8 | 2.4 |
| Rat Brain | 131 ± 5 | 216 ± 2 | 1.6 |

| | | | |
|---|---|---|---|
| * cpms of ¹⁴CO₂/µg protein/hr of triplicates ± S.E.M. | | | |

As shown in Table 2, bacterial lysates containing GAD₆₅ or GAD₆₇ catalyze the conversion of [1-¹⁴C]-glutamate to GABA and ¹⁴CO₂.

PLP stimulates the enzymatic activity of GAD₆₅ more than GAD₆₇ This greater stimulation probably reflects the faster cycling of GAD₆₅ through the inactivation cycle proposed by Martin and coworkers (Martin, *Cell.Mol.Neurobiol.,* 7:237, 1987). This faster cycling suggests that GAD₆₅ contributes more to the pool of apo-GAD that exists *in vivo* (Miller, *et al., Brain Res.Bull.,* 5(Suppl.2):89, 1980). Thus, *in vivo*, PLP appears to regulate GAD₆₅ activity more than GAD₆₇ activity.

GAD₆₅ activity in bacterial lysates is similar to the five-fold PLP stimulation of GAD activity found in synaptosomes prepared from rat substantia nigra (Miller, *et al., J.Neurochem.,* 33:533, 1979). Because both GADs are more dependent upon added PLP in bacteria than is the GAD activity in crude rat brain homogenates, the endogenous PLP concentration of bacteria lysates may be less than rat brain homogenates.

### D. IMMUNOLOGICAL IDENTIFICATION OF GAD₆₅ AND GAD₆₇

Rat brain homogenates and bacterial lysates were extracted as described above. Equal volumes of loading buffer were added to each sample as described (Harlow, *et al., Antibodies, A Laboratory Manual*, Cold Spring Harbor Laboratory, Cold Spring Harbor, NY, 1988). Proteins were separated by electrophoresis in a 10% acrylamide gel in SDS and electrophoretically transferred to nitrocellulose (Harlow, *et al., Antibodies, A Laboratory Manual,* Cold Spring Harbor Laboratory, Cold Spring Harbor, NY, 1988). The unreacted sites were blocked with a phosphate buffered saline (PBS) solution containing 2% bovine serum albumin (fraction V), 1% gelatin, and 1% Triton-X-100 at 42°C for one hr. After washing, the nitrocellulose filter was then cut into three sections and incubated with the following primary antibodies: lanes 1 to 4 with a 1/2000 dilution of the antiserum of Oertel, *et al.* (*Neuroscience*, 6:2689, 1981), which recognizes both GAD₆₇ and GAD₆₅; lanes 5-8 with a 1/2000 dilution of K-2 antiserum, which recognizes only GAD₆₇; lanes 9-12 with a 1/2000 dilution of GAD-6 monoclonal antibody, which is specific for GAD₆₅ (Chang, *et al., J.Neurosci*., 8:2123, 1988). All fitters were extensively washed and appropriate secondary antibodies were incubated and washed. Bound antibodies were detected with ¹²⁵I-labeled protein A and autoradiography. Each lane contained the following: lanes 1, 5, and 9 are BL21 (DE3) + pET-GAD₆₇; lanes 2, 6, and 10 are BL21(DE3) + pET-GAD₆₅; lanes 3, 7, and 11 are rat brain homogenate; and lanes 4, 8, and 12 are BL21 (DE3) + pET-8c.

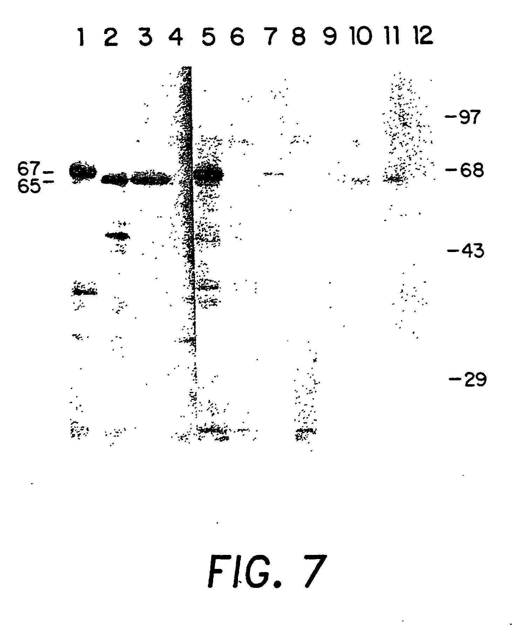

The immunoblots of bacterially produced GAD₆₅ and GAD₆₇ demonstrated that GAD₆₅ indeed corresponds to the smaller GAD in brain extracts, and GAD₆₇ to the larger form (Figure 7). Previous work has demonstrated the correspondence of GAD₆₇ to the larger GAD for feline GAD₆₇, and for mouse GAD₆₇ (Katarova, *et al., Eur.J.Neurosci.,* 2:190, 1990; 235, 1987). The mobilities of bacterially produced GAD₆₅ and GAD₆₇ (as detected with the antiserum of Oertel, *et al.* (*Neuroscience,* 6:2689, 1981) are identical to the immunoreactive doublet seen in rat brain homogenate.

The smaller molecular weight and larger molecular weight forms of GAD in rat brain are thus identical in antigenicity and size to the products of GAD₆₅ and GAD₆₇ cDNAs, respectively. Consequently, the two GADs in rat brain are GAD₆₅ and GAD₆₇. From this data it can also be concluded that the molecular identity of the reported PLP-dependent and PLP-independent GADs by Tapia (Bayon, *et al., J.Neurochem.*, 29:519, 1977) are GAD₆₅ and GAD₆₇, respectively. Martin and coworkers (Spink, *et al., Brain Res.,* 421:235, 1987) have reported the existence of four kinetically different forms of rat brain GAD. However, immunoblotting experiments (with the antisera used here) of these forms have not been reported.

### E. DISTRIBUTION OF GAD₆₅ and GAD₆₇ IN RNAs IN BRAIN TISSUE

Experiments were done to determine the distribution of GAD₆₅ and GAD₆₇ in RNAs in cerebellum using *in situ* hybridization.

Transcripts of, respectively, 3.2kb and 2.3kb from GAD₆₅ and GAD₆₇ cDNAs, were radiolabeled with ³⁵S according to Wuenschell, *et al.* (*Proc.Natl.Acad.Sci.,USA,* 83:6193, 1986) procedure. Hydrolyzed fragments of 200 bp were hybridized to coronal sections of a rat cerebellum. Animals were anesthetized under halothane and decapitated. The brain was rapidly frozen in dry ice and coronal frozen sections (12µm) were fixed for 30 min in freshly prepared 4% formaldehyde in phosphate-buffered saline (PBS; 130mM NaCl, 10mM Na phosphate, pH 7.0). The tissue was dehydrated through graded ethanol solutions and stored at -70°C.

In order to increase tissue permeability, the sections were submitted to the following pretreatments: rehydration through graded ethanol solutions (5 min each in 95%, 85%, 70%, 50%, and 30% ethanol); PBS (5 min); 0.02N HCl (10 min); PBS (5 min); 0.01% Triton N-101 in PBS (1 min); PBS (2 x 5 min); 1µg/ml proteinase K (7.5 min); and glycine (to inhibit proteinase K) in PBS (3 x 5 min). Proteinase K was digested for 30 min at 37°C before use. Sections were then incubated at 37°C in 50% formamide, 750mM NaCl, 25mM EDTA, 0.2% SDS, 0.02% BSA, 0.002% Ficoll, 0.02% polyvinylpyrrolidone, 250µg/ml yeast tRNA, 250µg/ml poly A, and 25mM PPES (pH 6.8).

For the hybridization, 100mM DTT, 10% dextran sulfate, and sense or antisense ³⁵S-RNA were added to the prehybridization solution. An aliquot (50µl) of the hybridization solution containing about 3 ng (10⁶ cpm) of probe (sense or antisense) was added onto the slides. Each slide was coverslipped and incubated for 16 hrs at 50°C, following which the siliconized coverslips were removed by brief washing in 4 x SSC (1 x SSC - 150mM NaCl, 60mM Na citrate, pH 7.0).

Sections were then treated with ribonuclease A (50µg/ml in 0.5M NaCl, 10mM Na thiosulfate, 1mM EDTA, 10mM TrisHCL, pH 8.0) for 20 min at 37°C and rinsed for 2 hrs at room temperature in 2 x SSC, 10mM Na thiosulfate, for 30 min at 55°C. Sections were dehydrated in ethanol, delipidated in xylene, coated with Kodak NTB2 emulsion and exposed for 10 days at 4°C. The emulsion was developed with Kodak D19, and the tissue counterstained with cresyl violet.

Autoradiographic grains were detected using reflected polarized light and grain numbers, densities, nd cell areas were determined with an Analytic Imaging Concepts image analyzer system. Due to the low background level, the criteria for defining a cell "labeled" was based on the presence of more than 5 clustered grains. The GAD labeled cells were found scattered throughout the brain, enabling the measurement of the number of grains over individual cells. The boundary of the cell and the area covered by a grain allowed the calculation of the number of grains per cell. This analysis was done at a high magnification (800X), using both reflected polarized light and transmitted light to simultaneously visualize the stained cell and the superimposed grains. Numbers are means ± S.E.M. of "n" cells.

**TABLE 3**

| | **GRAINS/CELL** | | |
|---|---|---|---|
| **CELL TYPE** | **GAD₆₇mRNA** | **GAD₆₅mRNA** | **GAD₆₇:GAD₆₅** |
| Purkinje | 172 ±34 (87)^{a} | 43 ± 2 (70) | 4.0 |
| Golgi II | 96 ± 8 (80) | 64 ± 9 (65) | 1.5 |
| Basket | 61 ± 12 (102) | 16 ± 1 (57) | 3.8 |
| Stellate | 55 ± 15 (65) | 18 ± 3 (37) | 3.1 |

| | | | |
|---|---|---|---|
| ^{a} ± S.E.M.(n) | | | |

In all neuronal types GAD₆₇ mRNA levels are greater. The observations with *in-situ* hybridization are consistent with previous findings (Nitsch, *J.Neurochem.,* 34:822, 1980; Denner, *et al.*, *J.Neurochem.*, 44:957, 1985; Itoh, *et al*., *Neurochem. Res.* 6:1283, 1981) that the ratio of PLP dependent to independent GAD activities in the cerebellum is one of the lowest in brain regions tested. In addition, as shown in Table 3, the order of amounts for GAD₆₇ mRNA is Purkinje > Golgi II > Basket > Stellate cells; in contrast, for GAD₆₅ mRNA, this order is Golgi II > Purkinje > Basket > Stellate cells.

The expression of GAD₆₅ and GAD₆₇ mRNAs thus differs among classes of neurons. The contribution of each to total GAD activity in turn affects how GABA production is regulated. For example, the substantia nigra contains one of the highest ratios of PLP-dependent to PLP-independent GAD activities (Nitsch, *J. Neurochem.*, 34:822, 1980). Increasing GABA concentration in the substantia nigra by local injection of inhibitors of GABA catabolism is especially effective in reducing seizure susceptibility (Gale, *Fed. Proc.*, 44:2414, 1985). Experimental animals undergoing seizures induced by PLP-antagonists may therefore be unable to inhibit seizure propagation because of inhibition of GAD₆₅ particularly in nerve terminals within the substantia nigra.

### F. SUBCELLULAR LOCATION OF GAD₆₅ AND GAD₅₇

The distribution of GAD₆₅ and GAD₆₇ was evaluated in the S₂ and synaptosome subcellular fractions. S₂ is a high speed supernatant consisting of the cytosol of all cells in the brain, while the synaptosomal fraction consists primarily of nerve endings (Gray, *et al., J. Anat, Lond,* 96:79, 1962). For these studies, whole rat brain fractionation was performed as described by Booth and Clark (Booth, *et al., Biochem. J.*, 176:365, 1978). Protein concentrations were determined by Schaffner and Weissman (Schaffner, *et al., Anal. Biochem.* 56:502, 1973). Samples were prepared as described (Kaiser, *et al., DNA Cloning, Vol. I, A Practical Approach*, *D.M. Glover ed.* (IRL Press, Oxford, 1985, pp. 38-40), and immunoblotting was done as described above using GAD-6 monoclonal antibody and K-2 antiserum. Equal amounts of protein (16µg) were added to each lane. Autoradiography showed a linear response of increasing amount of ¹²⁵l-protein A bound to antibody with protein concentrations of 1, 3, 10, 30, 100µgs with both K-2 antiserum and GAD-6 monoclonal antibody (data not shown).

The results showed that GAD₆₇ was present in equal amounts in both fractions. Since the S₂ fraction contains the cytosolic proteins of glial (as well as other non-neuronal) and neuronal cells, the concentration of GAD₆₇ must be greater in neuronal cell bodies than in nerve endings. In contrast, the concentration of GAD₆₅ was greater in synaptosomes than in S₂. These subcellular fractionation experiments suggest that, in contrast to GAD₆₅, a much greater fraction of GAD₆₇ s present in cell bodies of neurons than in nerve terminals. Thus, subcellular fractionation, like immunohistochemistry, shows that GAD₆₅ and GAD₆₇ have different subcellular distributions.

*In vivo* experiments utilizing inhibitors of GABA synthesis and degradation have suggested that the GABA pool in neuronal cell bodies is different from that in the nerve terminals (ladarola, *et al., Mol. Cell. Biochem,* 39:305, 1981). GABA produced by GAD₆₇ may be involved more in cellular metabolism (for example, in the GABA shunt) and in dendrodendritic synapses. The dendrites of granule cells in the olfactory bulb, which form dendrodendritic synapses with mitral dendrites (Shepard, *Physiol. Rev.*, 52:864, 1972) and probably release GABA (McLennan, *Brain Res.,* 29:177-184, 1971), label intensely with K-2 antiserum. While not shown here, it has also been found greater GAD₆₇ than GAD₆₅ mRNA levels (2-3 fold) in the olfactory bulb. This distribution is consistent with the reported finding that most GAD activity in the olfactory bulb is present in S₂ and P₁ (crude nudear pellet) and not in synaptosomes (Quinn, *et al., J. Neurochem*., 35:583, 1980).

The differing subcellular distributions of GAD₆₅ and GAD₆₇ could result from cytoskeletal anchoring or from some unknown protein targeting mechanism. Some cytoskeletal proteins have distributions that resemble GAD₆₅ and GAD₆₇, For example, in cultured sympathetic neurons Peng, *et al*. *(J Cell. Biol.,* 102:252. 1986), demonstrate that 84% of tau is in axons while 100% of MAP-2 is in cell bodies and dendrites. In addition, 43kd protein, a cytoskeletal protein, is thought to anchor the acetylcholine receptor to the underlying membrane cytoskeleton (Flucher, *et al., Neuron,* 3:163, 1989).

### EXAMPLE 3

### DETECTION OF GAD AUTOANTIBODIES IN CLINICAL SPECIMENS

### A. MATERIALS AND METHODS

**1. Patient Specimens**. Sera from four groups of individuals were selected from a previous study by Atkinson and co-workers (Atkinson, *et al., Lancet*, 335:1357-1360, 1990). These groups consisted of: Group (1), 1 new onset IDD patients diagnosed according to the established National Diabetes Data Group (NDDG) criteria (Gleichman, *et al., Diabetes,* 36:578-584, 1987) that had been referred to the University of Florida, Diabetes Clinics; Group (2), 5 randomly selected islet cell cytoplasmic antibody (ICA) negative non-diabetic controls without any known family history of autoimmune disease; Group (3), 13 individuals whose sera had been collected 3 to 66 months prior to their documented clinical onsets of IDD; Group (4), non-diabetic controls and relatives, and those who were studied prior to their onsets of IDD; and Group (5), 3 patients at risk for IDDM, but where onset has not yet occurred. This latter group had been ascertained through ongoing prospective ICA screening studies of more than 5000 first degree relative of IDD probands, and 8200 individuals from the general population (of which 4813 were school children).
**2. Islet Cell Autoantibodies.** ICA were assayed by indirect immunofluorescence on blood group O cryocut pancreatic (Atkinson, *et al., Lancet,* 335:1357-1360, 1990). All results were interpreted on coded samples, with control negative and positive sera in each batch. The degrees of ICA positivity were analyzed with the guidelines established by the Immunology Diabetes Workshop (IDW) for the standardization of ICA (Gleichman, *et al., Diabetes*, 36:578-584, 1987). All positive sera were titered by end point dilution, and the Juvenile Diabetes Foundation (JDF) units were determined by reference to a standard serum previously calibrated to the international JDF standard of 80 units. In the studies reported here, a positive ICA result was defined by replicate titers of 10 JDF units or greater.
**3. HLA DR Typing.** HLA DR typing was performed as adapted from the method described by Van Rood and Van Leuwen (*Nature,* 262:795-797, 1976), using DR trays (One Lamda Laboratories, Los Angeles, CA).
**4. Human Islet Cells**. Human pancreatic islets were isolated from cadaveric pancreases and maintained *in vitro* as previously described (Ricordi, *et al., Diabetes*, 37:413-420, 1988). The islet cells were metabolically labeled with ³⁵S methionine (Amersham, Arlington Heights, IL) *in vitro* (95% air/5%CO₂).
**5. Islet Cell Extractions and Immunoprecipitations.** Islet cells were extracted as previously described by Atkinson, *et al.* (*Lancet,* 335:1357-1360, 1990) with the following modifications. For immunoprecipitation studies, the islet cell lysates were precleared twice by incubation (2h, 4°C) with either control, IDD serum (100µl), or GAD-6 (Chang, *et al., J.Neuro*, 8:2123-2130, 1988) (1µl in 99µl of Tris buffer (Atkinson, *et al., Lancet*, 335:1357-1360, 1990) for every 1000 islets. Immune complexes were then absorbed (1h 4°C) with an excess of protein A Sepharose CL-4B (Pharmacia, NJ). Aliquot volumes representing 1000 islet calls containing unbound (precleared) lysate were then incubated (12h, 4°C) with either IDD or control sera (25µl), or GAD-6 (Chang, *et al., J.Neuro*, 8:2123-2130, 1988) (1 µl in 25µl Tris buffer). Following another incubation with protein A Sepharose CL-4B (1h, 4°C), the complexes were then washed 5 times with 50mM Tris HCL (pH 7.4) with 0.1% SDS, 1.0% Triton X-114, and 2mM EDTA, and then washed again one time in double distilled water. The protein A Sepharose CL-4B was then boiled in Laemmli sample buffer (Laemmli, *Nature,* 227:680-685, 1970), and the samples were subjected to SOS-PAGE and fluororadiography (Kodak, X-omat AR5) using Enhance (New England Nuclear). Alternatively, the autoradiographs were analyzed by a BETAGEN (Boston, MA) analyzer. Both 64KA positive and negative sera were used in each assay, to serve as interassay controls. All fluororadiographs were analyzed and rated as positive or negative after comparison with the known interassay controls. Positive serum samples were designated as 1 when a sample resulted in immunoprecipitation of a low intensity 64,000 Mₓ band, 2 if a moderate intensity band was observed and 3 if the intensity of the immunoprecipitated protein was high. A similar rating procedure was employed for the intensity of bands corresponding to immunoprecipitated ³⁵S-GAD₆₅ and ³⁵S-GAD₆₇.
**6. Immunoprecipitations.** Immunoprecipitation of bacterial lysates containing ³⁵S-GAD₆₅ or ³⁵S-GAD₆₇, and GAD from human brain homogenate, was completed as described above in immunoprecipitation studies of human islet cell extractions.
**7. GAD Assays.** Human brain homogenates were incubated with patient sera as described above in human islet cells. After absorption and washes, the protein A agarose slurry was aliquoted into three equal volumes and GAD activity was measured as described (Krieger, *et al., Neurochem.* 33:299, 1984). Briefly, Protein A agarose beads were incubated with (1-¹⁴C)-glutamate (Amersham) in a designated incubation mixture (Krieger, *et al., Neurochem*. 33:299, 1984) and production of ¹⁴CO₂ was quantitated by a liquid scintillation counter.
**8. Production of ³⁵S-GAD₆₅ and ³⁵S-GAD₆₇** Rat GAD₆₅ and GAD₆₇ cDNAs were subdoned into a bacterial expression system as previously described. Labeling of ³⁵S-GADs was completed by pulsing IPTG induced bacterium (growing in Minimal Media) for 15 minutes with TRAN ³⁵S-label (ICN). Cultures were then spun down and resuspended and sonicated in 1 ml of homogenizing buffer (1mM phenylmethylsulfonyl fluoride (PMSF), 1 mM 2-aminoethylisothiouronium Bromide (AET) and 60mM potassium phosphate, pH 7.1). After sonication, cell debris was removed by centrifugation and protein concentration was measured (Bradford, *Anal. Biochem.,* 72:248. 1986) in the supernatant (supematant was stored in aliquots at -70°C).

### B. IMMUNOREACTIVITY OF IDDM SPECIMENS

Sera from patients with IDDM were tested for the ability to precipitate GAD from human brain homogenates.

**TABLE 4**

| SERA FROM IDDM PATIENTS IMMUNOPRECIPITATE GAD ACTIVITY | | | | | |
|---|---|---|---|---|---|
| Patient | IDDM | Pre-IDDM Perlod¹ | 64K² | JDF³ | GAD Activity⁴ cpm's |
| DA | *⁵ | >24 | 3 | 164 | 13,762 |
| DC | * | >1 | 3 | 20 | 1.719 |
| RS | + | 5 | 3 | 40 | 588 |
| III. | + | 0 | 2 | 80 | 440 |
| DM | * | >1 | 2 | 10 | 184 |
| c | - | na | 0 | 0 | 280 |
| c | - | na | 0 | 0 | 285 |
| c | - | na | 0 | 0 | 325 |
| c | - | na | 0 | 0 | 275 |
| c | - | na | 0 | 0 | 270 |

| | | | | | |
|---|---|---|---|---|---|
| ¹ Expressed an months ² 64K liters as described in Experimental Methods ³ The islet cell antibody test as expressed in Juvenile Diabetes Foundation (JDF) units ⁴ Hot adjusted for background ⁵ At risk for diabetes (also, failed glucose test) na - Hot applicable | | | | | |

As shown in Table 4, the sera of four (out of five) at risk for IDDM or IDDM patients bound significantly greater amounts of enzymatically active GAD of human brain extracts than sera from control patients. In addition sera from one of the patients was drawn in a pre-IDDM period, thus autoantibodies to GAD are present prior to the onset of IDDM symptoms (see C below).

Further experiments (results not presented) showed that the sera of two at risk IDDM patients (DA, DC) immunoprecipitated recombinantly produced ³⁵S-GAD₆₅ whereas recombinantly produced ³⁵S-GAD₆₇ was only recognized by sera of patient DA (and to a lesser degree than ³⁵S-GAD₆₅). Subsequent studies have found larger titers of GAD₆₇ autoantibodies than GAD₆₅ are present in sera of IDDM patients with neuropathic complications (not shown here).

Additional studies using patient DA sera showed the presence of antibodies which recognize specific polypeptides produced in human pancreatic islet cells. Electrophoretic analysis of the bound polypeptides demonstrated the presence of autoantibodies to a 64kD component, as previously shown by others in human IDDM (Baekkeskov, *et al., Nature,* 298:167-169, 1982) and in animal models (Baekkeskov, *et al., Science,* 224:1348-1350, 1984; Atkinson, *et al., Diabetes,* 37:1587-1590, 1988). Prior absorption of these sera with GAD-6 monoclonal, which recognized GAD₆₅ but not GAD₆₇, or with bacterially produced GAD₆₅, abolished the ability of the sera to recognize the 64kD pancreatic polypeptide. The epitopes recognized by autoantibodies to the 64kD autoantigen are thus present in GAD₆₅, indicating that the autoantigen is indeed GAD₆₅. In order to investigate the predictive value of GAD₆₅, sera drawn from patients prior to onset of clinical manifestation of IDDM were tested for autoantibodies to GAD₆₅.

**TABLE 5.**

| IPPM PATIENTS ANALYZED FOR AUTOANTIBODIES PRIOR TO THE ONSET OF DISEASE | | | | | | | | |
|---|---|---|---|---|---|---|---|---|
| Patient | Sex | IIIA | Age Onset¹ | Pre-IDD Period² | JDF | 64KA³ | GAD²₆₅ | GDA¹₆₇ |
| TA | H | 3,2 | 17 | 11 | 20 | 2 | 0 | 1 |
| CA | F | 4,5 5 | 38 | 4 | 0 | 1 | 1 | 0 |
| RA | H | 2,1 | 5 | 34 | 0 | 2 | 1 | 0 |
| TB | H | 2,4 | 11 | 66 | 40 | 1 | 1 | 0 |
| AB | H | N.D. | 23 | 6 | 160 | 3 | 3 | 2 |
| VC | F | 4,6 | 15 | 3 | 40 | 1 | 0 | 1 |
| JD | H | 6,1 | 34 | 25 | 10 | 3 | 1 | 1 |
| DR | F | 3,4 | 14 | 42 | 320 | 2 | 1 | 0 |
| JG | H | 3,3 | 12 | 8 | 40 | 1 | 0 | 0 |
| BR | H | 3,3 | 5 | 9 | 0 | 0 | 1 | t |
| KR | F | 4,X | 34 | 14 | 10 | 3 | 2 | 0 |
| JT | F | 4,6 | 7 | 10 | N.D. | 1 | 1 | 1 |

| | | | | | | | | |
|---|---|---|---|---|---|---|---|---|
| ¹ Age of IDDH onset expressed as months ² The time Interval between sera drawn and IDDM onset expressed as months ³ 1 - lowest; 2 - medium; and 3 - highest band intensities N.D. - not determined | | | | | | | | |

As shown in Table 5, 9 out of 12 specimens (75%) were immunoreactive with ³⁵S-GAD₆₅. In addition, two patients (JA and VC) were immunoreactive to GAD₆₇, but not GAD₆₅ under these conditions. Therefore, in combination, autoantibodies to GAD₆₅ and GAD₆₇ were present in 11 out of 12 (91%) of these patients sera. This finding suggests that although autoantibodies to GAD₆₅ are more common than autoantibodies to GAD₆₇, the use of both recombinant GADs (GAD₆₅ and GAD₆₇) in an assay would allow for greater predictability of IDDM. Previous tests of these sera (Atkinson, *et al*., *Langet*, 335:1357-1360, 1990) demonstrated that 11 out of 12, or 92%, immunoreacted with the ³⁵S-64kD molecule from human pancreatic islet cells. The serum which contained detectable autoantibodies to the 64kD molecule and not GAD₆₅ was a serum which contained the lowest titer (or "1") for the 64kD molecule. Thus, the false negative obtained was due to a lack of sensitivity in this assay. Furthermore, this assay predicted IDDM in one patient (BR) who was negative for 64K.

These results show that the 64kD molecule identified in β-cells of human pancreas is identical in size and antigenicity to rat GAD₆₅. Furthermore, sera drawn from patients prior to IDDM onset contain autoantibodies to GAD₆₅. Consequently, the GAD₆₅ recombinant molecule is of great utility as a diagnostic tool for predicting IDDM. The ability of a physician to diagnose IDDM prior to actual symptoms will no doubt result in a greater extension of time before insulin therapy is needed. The sensitivity of such immunoassays will improve with the use of a recombinant GAD₆₅ of human origin which represents the GAD form present in β-cells of the pancreas.

### EXAMPLE 4

### IMMUNE PROLIFERATIVE RESPONSE TO POLYPEPTIDE

Polypeptides were synthesized using an automatic instrument (Applied Biosystems) and standard conditions. These polypeptides were then tested to compare their relative ability to stimulate proliferation of splenic lymphocytes and islet infiltrating T lymphocytes (IITLs). In this study, polypeptides derived from the GAD₆₅ core sequence and from the homologous region of polio virus were compared. Appropriate cells were cultured for 5 days with the respective polypeptide in the presence of 5 X 10⁴ irradiated spleen cells. ³H-thymidine was added during the last 16 hours of culture.

**TABLE 6**

| **ANTIGEN** | **AMINO ACID SEQUENCE** | **³H-THYMIDINE INCORPORATION (cpm) BY LYMPHOID CELL POPULATION** | |
|---|---|---|---|
| | | **IITLs^{a}** | **SPLEEN^{b}** |
| None | - | 1,100 | 6,500 |
| Poliovirus | MKSMCPCAQLKVKYL | 900 | 22,500 |
| GAD₆₅ | ARFKMFPEVKEKGMAA | 9,500 | 23,300 |

| | | | |
|---|---|---|---|
| ^{a} islet infiltrating T lymphocytes (3 X 10⁴ cells/well) ^{b} 1 X 10⁵ cells/well | | | |

In these studies, there was no significant difference in the proliferative activity of cultures of spleen lymphocytes exposed to either the polio or the GAD₆₅ polypeptides. However, both polypeptides stimulated a T cell response which was higher than that found in the media control. The lack of difference in proliferation in the spleen cell population may be due to a lower frequency of GAD polypeptide specific T cells.

The llTL population, when evaluated in the same manner, showed a marked difference in cell proliferation. In this system, the response to the GAD₆₅ polypeptide was 9-fold greater than that of either the culture media or the polio polypeptide. This data strongly suggests that the GAD₆₅ is an important antigen for T cell responses in the IITL population. This data suggests that molecular mimicry plays a role in the pathogenesis of diabetes.

## Claims

1. Use of a polypeptide or analog, chemical derivative, or pharmaceutically acceptable salt thereof having the following amino acid sequence:
X-Pro-Glu-Val-Lys-Y-Lys-Z,
wherein X is an amino acid sequence selected from one to ten amino acids or omitted; Y is Thr or Glu; and Z is an amino acid sequence selected from one to eight amino acids or omitted
(a) for diagnosing insulin-dependent diabetes mellitus (IDDM);
(b) for detecting antibodies to GAD₆₅ in a sample;
(c) for classifying patients with autoimmune diseases;
(d) for screening drugs that alter GAD function;
(e) for the generation of an antibody;

2. The use of claim 1, wherein X comprises Lys, Y is Thr, and Z comprises Leu.

3. The use of claim 1, wherein X comprises Lys, Y is Glu, and Z comprises Leu.

4. The use of claim 3, wherein X further comprises Met, and Z further comprises Arg and Leu.

5. The use of any one of claims 1 to 3, wherein Xis the amino acid sequence Ser - Ile - Met - Ala - Ala - Arg - Tyr - Lys - Tyr - Phe, and Z is the amino acid sequence Gly - Met - Ala - Ala - Val - Pro - Lys - Leu.

6. The use of any one of claims 1 to 4, wherein X is the amino acid sequence Ala - Met - Met - Ile - Ala - Arg - Phe - Lys - Met - Phe, and Z is the amino acid sequence Gly - Met - Ala - Ala - Leu - Pro - Arg - Leu.

7. The use of any one of claims 1(b), or 2 to 6, wherein said antibodies are autoantibodies.

8. The use of any one of claims 1 (c), or 2 to 6, wherein said autoimmune disease is insulin-dependent diabetes mellitus (IDDM).

9. The use of any one of claims 1 (e), or 2 to 6, wherein the antibody is polyclonal or monoclonal.

10. The use of any one of claims 1(b), or 2 to 7, wherein the polypeptide is employed in a competitive or non-competitive immunoassay in a direct or indirect format.

11. The use of claim 10, wherein said immunoassay is a radio immunoassay (RIA), a sandwich immunometric assay, or a western blot assay.

12. The use of any one of claims 1(b), 2 to 7, or 10, wherein antibodies to GAD₆₅ are detected by measuring GAD enzymatic activity.

13. Use of an antibody to the polypeptide as defined in any one of the preceding claims for the detection of GAD₆₅.

14. The use of claim 13, wherein said antibody is monoclonal.

15. The use of claim 13 or 14, wherein said antibody is bound to a lectin, preferably ricin.

16. A pharmaceutical composition comprising the polypeptide as defined in any one of the preceding claims, wherein the polypeptide is labeled.

17. The pharmaceutical composition of claim 16, wherein the polypeptide is labeled with a therapeutic agent or a radioisotope.

## Patentansprüche

1. Verwendung eines Polypeptids oder Analogs, eines chemischen Derivats oder eines pharmazeutisch verträglichen Salzes davon, welches folgende Aminosäuresequenz hat:
X-Pro-Glu-Val-Lys-Y-Lys-Z,
wobei X eine Aminosäuresequenz ausgewählt aus einer bis zehn Aminosäuren ist oder weggelassen wird; Y Thr oder Glu ist; und Z eine Aminosäuresequenz ist ausgewählt aus einer bis acht Aminosäuren oder weggelassen wird
(a) zur Diagnose von insulinabhängigem Diabetes mellitus (IDDM);
(b) zum Nachweis von Antikörpern gegen GAD₆₅ in einer Probe;
(c) zur Klassifikation von Patienten mit Autoimmunerkrankungen;
(d) zum Screenen nach Arzneistoffen, welche die GAD-Funktion ändern;
(e) zur Erzeugung eines Antikörpers.

2. Verwendung nach Anspruch 1, wobei X Lys umfasst, Y Thr ist und Z Leu umfasst.

3. Verwendung nach Anspruch 1, wobei X Lys umfasst, Y Glu ist und Z Leu umfasst.

4. Verwendung nach Anspruch 3, wobei X ferner Met umfasst und Z ferner Arg und Leu umfasst.

5. Verwendung nach einem der Ansprüche 1 bis 3, wobei X die Aminosäuresequenz Ser - Ile - Met - Ala - Ala - Arg - Tyr - Lys - Tyr - Phe ist und Z die Aminosäuresequenz Gly - Met - Ala - Ala - Val - Pro - Lys - Leu ist.

6. Verwendung nach einem der Ansprüche 1 bis 4, wobei X die Aminosäuresequenz Ala - Met - Met - Ile - Ala - Arg - Phe - Lys - Met - Phe ist und Z die Aminosäuresequenz Gly - Met - Ala - Ala - Leu - Pro - Arg - Leu ist.

7. Verwendung nach einem der Ansprüche 1 (b) oder 2 bis 6, wobei die Antikörper Autoantikörper sind.

8. Verwendung nach einem der Ansprüche 1(c) oder 2 bis 6, wobei die Autoimmunerkrankung insulinabhängiger Diabetes mellitus (IDDM) ist.

9. Verwendung nach einem der Ansprüche 1 (e) oder 2 bis 6, wobei der Antikörper ein polyclonaler oder monoclonaler Antikörper ist.

10. Verwendung nach einem der Ansprüche 1(b) oder 2 bis 7, wobei das Polypeptid in einem kompetitiven oder nicht-kompetitiven Immunoassay in einem direkten oder indirekten Format eingesetzt ist.

11. Verwendung nach Anspruch 10, wobei der Immunoassay ein Radioimmunoassay (RIA), ein immunometrischer Assay vom Sandwichtyp oder ein Western-Blot-Assay ist.

12. Verwendung nach einem der Ansprüche 1(b) oder 2 bis 7 oder 10, wobei die Antikörper gegen GAD₆₅ durch Messung der GAD-Enzymaktivität nachgewiesen werden.

13. Verwendung eines Antikörpers gegen das Polypeptid wie in einem der vorangegangenen Ansprüche definiert zum Nachweis vom GAD₆₅.

14. Verwendung nach Anspruch 13, wobei der Antikörper ein monoclonaler Antikörper ist.

15. Verwendung nach Anspruch 13 oder 14, wobei der Antikörper an ein Lectin, vorzugsweise Ricin, gebunden ist.

16. Arzneimittel umfassend das Polypeptid wie in einem der vorangegangenen Ansprüche definiert, wobei das Polypeptid markiert ist.

17. Arzneimittel nach Anspruch 16, wobei das Polypeptid mit einem therapeutischen Mittel oder einem Radioisotop markiert ist.

## Revendications

1. Utilisation d'un polypeptide ou d'un de ses analogues, dérivés chimiques ou sels pharmaceutiquement acceptables, ayant la séquence d'aminoacides suivante:
X-Pro-Glu-Val-Lys-Y-Lys-Z
dans laquelle X est une séquence d'aminoacides choisie de 1 à 10 aminoacides ou est omis; Y est Thr ou Glu; et Z est une séquence d'aminoacides choisie de 1 à 8 aminoacides ou est omis
(a) pour le diagnostic du diabète insulino-dépendant (DID);
(b) pour la détection d'anticorps dirigés contre la GAD₆₅ dans un échantillon;
(c) pour la classification de patients ayant des maladies auto-immunes;
(d) pour le criblage de médicaments qui altèrent la fonction de la GAD;
(e) pour la production d'un anticorps.

2. Utilisation selon la revendication 1, dans laquelle X comprend Lys, Y est Thr et Z comprend Leu.

3. Utilisation selon la revendication 1, dans laquelle X comprend Lys, Y est Glu et Z comprend Leu.

4. Utilisation selon la revendication 3, dans laquelle X comprend en outre Met, et Z comprend en outre Arg et Leu.

5. Utilisation selon l'une quelconque des revendications 1 à 3, dans laquelle X est la séquence d'aminoacides Ser - Ile - Met - Ala - Ala - Arg - Tyr - Lys - Tyr - Phe, et Z est la séquence d'aminoacides Gly - Met - Ala - Ala - Val - Pro - Lys - Leu.

6. Utilisation selon l'une quelconque des revendications 1 à 4, dans laquelle X est la séquence d'aminoacides Ala - Met - Met - Ile - Ala - Arg - Phe - Lys - Met - Phe, et Z est la séquence d'aminoacides Gly - Met - Ala - Ala - Leu - Pro - Arg - Leu.

7. Utilisation selon l'une quelconque des revendications 1 (b) ou 2 à 6, dans laquelle lesdits anticorps sont des auto-anticorps.

8. Utilisation selon l'une quelconque des revendications 1(c) ou 2 à 6, dans laquelle ladite maladie auto-immune est le diabète insulino-dépendant (DID).

9. Utilisation selon l'une quelconque des revendications 1 (e) ou 2 à 6, dans laquelle l'anticorps est polyclonal ou monoclonal.

10. Utilisation selon l'une quelconque des revendications 1(b) ou 2 à 7, dans laquelle le polypeptide est utilisé dans une analyse immunologique compétitive ou non compétitive dans un format direct ou indirect.

11. Utilisation selon la revendication 10, dans laquelle l'analyse immunologique est une analyse radio-immunologique (RIA), une analyse immunométrique en sandwich ou une analyse par transfert western.

12. Utilisation selon l'une quelconque des revendications 1(b), 2 à 7 ou 10, dans laquelle les anticorps dirigés contre la GAD₆₅ sont détectés par mesure de l'activité enzymatique de GAD.

13. Utilisation d'un anticorps dirigé contre le polypeptide tel que défini dans l'une quelconque des revendications précédentes pour la détection de la GAD₆₅.

14. Utilisation selon la revendication 13, dans laquelle ledit anticorps est monoclonal.

15. Utilisation selon la revendication 13 ou 14, dans laquelle ledit anticorps est lié à une lectine, de préférence la ricine.

16. Composition pharmaceutique comprenant le polypeptide tel que défini dans l'une quelconque des revendications précédentes, dans laquelle le polypeptide est marqué.

17. Composition pharmaceutique selon la revendication 16, dans laquelle le polypeptide est marqué avec un agent thérapeutique ou un isotope radioactif.
